# FASCICULE DE BREVET EUROPEEN

(11) **EP 1 757 586 B1**
(45) Date de publication et mention de la délivrance du brevet: **03.12.2008**
(21) Numéro de dépôt: 06291218.3
(22) Date de dépôt: 27.07.2006
(51) Int. Cl.: C07D 213/74, A61K 31/44

(54) **Nouveaux composés 1,1-pyridinylaminocyclopropanamines polysubstitués, leur procédé de préparation et les compositions pharmaceutiques yui les contiennent**
NOVEL POLYSUBSTITUTED 1,1-PYRIDINYLAMINOCYCLOPROPANAMINE DERIVATIVES, PROCESSES FOR THEIR PREPARATION AND PHARMACEUTICAL COMPOSITION CONTAINING THEM
POLYSBUSTITUIRTE 1,1-PYRIDINYLAMINOCYCLOPROPANAMIN DERIVATE, VERFAHREN ZU DEREN HERSTELLUNG UND DIESE ENTHALTENDE PHARMATUETISCHE ZUSAMMENSETZUNG

(30) Priorité: 28.07.2005 FR 0508032
(43) Date de publication de la demande: 28.02.2007
(73) Titulaire: Les Laboratoires Servier, 92415 Courbevoie Cedex (FR)
(72) Inventeur: Goldstein, Solo, 92150 Suresnes (FR); Guillonneau, Claude, 92140 Clamart (FR); Charton, Yves, 92330 Sceaux (FR); Lockhart, Brian, 78810, Feucherolles (FR); Lestage, Pierre, 78170 La Celle Saint Cloud (FR)

(56) Documents cités:
- EP-A- 1 170 281
- WO-A-00/75110
- WO-A-01/70733
- WO-A-01/87288
- WO-A-99/21834

## Description

La présente invention concerne de nouveaux composés 1,1-pyridinylamino-cyclopropanamines polysubstitués, leur procédé de préparation et les compositions pharmaceutiques qui les contiennent.

Les composés de la présente invention sont particulièrement intéressants d'un point de vue pharmacologique pour leur interaction spécifique avec les récepteurs nicotiniques centraux de type α4β2, trouvant leur application dans le traitement des neuropathologies associées au vieillissement cérébral, des troubles de l'humeur, de la douleur et du sevrage tabagique.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence. Il est largement démontré que parmi les différents neurotransmetteurs, l'acétylcholine tient une place prépondérante dans les fonctions de mémoire et que les voies neuronales cholinergiques sont dramatiquement détruites lors de certaines maladies neurodégénératives ou en déficit d'activation lors du vieillissement cérébral. C'est pourquoi, de nombreuses approches thérapeutiques ont visé à empêcher la destruction du neuromédiateur *via* l'inhibition de l'acétylcholine-estérase ou ont cherché à se substituer au neuromédiateur déficitaire. Dans ce dernier cas, les agonistes cholinergiques proposés ont été de type muscarinique, spécifiques pour les récepteurs post-synaptiques M1.

Récemment, il a été montré que l'atteinte cholinergique liée à la maladie d'Alzheimer touchait davantage les neurones portant les récepteurs nicotiniques que ceux portant les récepteurs muscariniques (Schroder et Coll., « Alzheimer disease : therapeutic strategies », Birkhauser Boston, 1994, 181-185). De plus, de nombreuses études ont démontré que la nicotine possède des propriétés facilitatrices de la mémoire (Prog. Neuropsychopharmacol., 1992, 16, 181-191) et que ces propriétés s'exercent tout autant sur les fonctions mnésiques (Psychopharmacol., 1996, 123, 88-97) que sur les facultés d'attention et de vigilance (Psychopharmacol., 1995, 118, 195-205). Par ailleurs, la nicotine exerce des effets neuroprotecteurs vis-à-vis d'agents excitotoxiques tel que le glutamate (Brain Res., 1994, 644, 181-187).

L'ensemble de ces données est très probablement à relier avec les études épidémiologiques qui ont montré une moindre incidence de maladie d'Alzheimer ou de Parkinson chez les sujets fumeurs. De plus, plusieurs études ont montré l'intérêt de la nicotine dans le traitement des troubles de l'humeur tels que les états dépressifs, anxieux ou schizophréniques. Enfin, il a été montré que la nicotine possède des propriétés antalgiques. L'ensemble des propriétés thérapeutiques de la nicotine, ainsi que celles décrites pour d'autres agents nicotiniques, est sous tendu par une activité vis-à-vis de récepteurs centraux qui diffèrent structurellement et pharmacologiquement des récepteurs périphériques (muscle et ganglion). Les récepteurs centraux de type α4β2 sont les plus représentés dans le système nerveux central et ont été impliqués dans la plupart des effets thérapeutiques de la nicotine (Life Sci., 1995, 56, 545-570).

Plusieurs documents tels que Synlett., 1999, 7, 1053-1054 ; J. Med. Chem, 1985, 28(12), 1953-1957 et 1980, 23(3), 339-341 ; 1970, 13(5), 820-826 ; 1972, 15(10), 1003-1006 ; J. Am. Chem. Soc., 1987, 109(13), 4036-4046, ou quelques brevets ou demandes de brevets comme DE 36 08 727, EP 124 208 ou WO 94/10158 décrivent et revendiquent des composés comportant un motif cyclopropanique 1,1 ou 1,2-disubstitué. Aucunes de ces références ne décrivent ou ne suggèrent pour ces composés une activité pharmacologique spécifique vis-à-vis des récepteurs nicotiniques et plus particulièrement vis-à-vis des récepteurs nicotiniques centraux de type α4β2, propriété originale des composés décrits par la Demanderesse. La demande de brevet EP 1 170 281 décrit des composés cyclopropaniques 1,1 et 1,2-disubstitués qui sont des ligands nicotiniques.

Les composés de la présente invention sont donc nouveaux et constituent de puissants ligands nicotiniques sélectifs du sous-type réceptoriel α4β2 central. De ce fait, ils sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

Plus particulièrement, la présente invention concerne les composés de formule (I) : 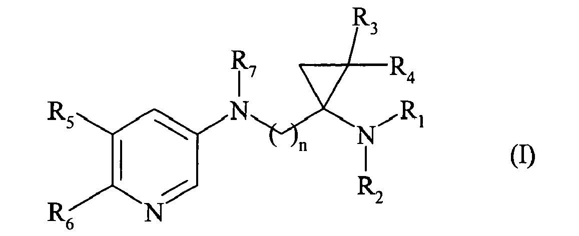 dans laquelle :
n représente un nombre entier compris entre 1 et 6 inclus,
R₁ et R₂, identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
R₃ et R₄, identiques ou différents, indépendamment l'un de l'autre, représentent un atome d'hydrogène, ou un groupement alkyle (C₁-C₆) linéaire ou ramifié,
R₅ et R₆, identiques ou différents, indépendamment l'un de l'autre, représentent un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, ou amino éventuellement substitué par un ou deux groupements alkyle (C₁-C₆) linéaire ou ramifié,
R₇ représente un atome d'hydrogène, ou un groupement alkyle (C₁-C₆) linéaire ou ramifié ou arylalkyle (C₁-C₆) linéaire ou ramifié,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié.

Des composés préférés de l'invention sont les composés pour lesquels n est un entier prenant la valeur 1.

Les substituants R₁ et R₂ préférés selon l'invention sont l'atome d'hydrogène et le groupement alkyle (C₁-C₆) linéaire ou ramifié.

Encore plus préférentiellement, les substituants R₁ et R₂ préférés selon l'invention sont l'atome d'hydrogène et le groupement méthyle.

Les substituants R₃ et R₄ préférés selon l'invention sont l'atome d'hydrogène.

Les substituants R₅ et R₆ préférés selon l'invention sont l'atome d'hydrogène, le groupement alkyle (C₁-C₆) linéaire ou ramifié et l'atome d'halogène.

D'une façon avantageuse, les composés préférés de l'invention sont les composés pour lesquels R₅ représente un atome d'hydrogène et R₆ représente un groupement alkyle (C₁-C₆) linéaire ou ramifié ou un atome d'halogène.

D'une façon encore plus avantageuse, les composés préférés de l'invention sont les composés pour lesquels R₅ représente un atome d'hydrogène et R₆ représente un groupement méthyle ou un atome d'halogène.

Le substituant R₇ préféré selon l'invention est l'atome d'hydrogène et le groupement alkyle (C₁-C₆) linéaire ou ramifié.
D'une façon avantageuse, le substituant R₇ préféré selon l'invention est l'atome d'hydrogène et le groupement méthyle.

D'une façon particulièrement avantageuse, les composés préférés de l'invention sont le :
*N*-{[1-(méthylamino)cyclopropyl]méthyl}pyridin-3-amine
*N*-méthyl-*N*-{[1-(méthylamino)cyclopropyl]méthyl}pyridin-3-amine
*N*-[(1-aminocyclopropyl)méthyl] pyridin-3-amine
*N*-{[1-(diméthylamino)cyclopropyl]méthyl}pyridin-3-amine
*N*-[(1-aminocyclopropyl)méthyl]-*N*-méthylpyridin-3-amine
*N-*{[1-(diméthylamino)cyclopropyl]méthyl}-*N*-méthylpyridin-3-amine
6-chloro-*N*- {[1-(méthylamino)cyclopropyl]méthyl}pyridin-3-amine
6-chloro-*N*-méthyl-*N*-{[1-(méthylamino)cyclopropyl]méthyl}pyridin-3-amine
6-bromo-*N*-{[1-(méthylamino)cyclopropyl]méthyl}pyridin-3-amine
6-bromo-*N*-méthyl-*N*-{[1-(méthylamino)cyclopropyl]méthyl}pyridin-3-amine
6-méthyl-N- {[1-(méthylamino)cyclopropyl]méthyl}pyridin-3-amine
*N*,6-diméthyl-*N*-{[1-(méthylamino)cyclopropyl]méthyl}pyridin-3-amine

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les énantiomères, diastéréoisomères, ainsi que les sels d'addition à un acide ou à une base pharmaceutiquement acceptable des composés préférés font partie intégrante de l'invention.

La présente invention concerne également le procédé de préparation des composés de formule (I), caractérisé en ce qu'on utilise comme produit de départ un composé de formule (II) : 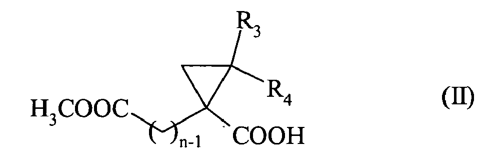 dans lequel R₃, R₄ et n sont tels que définis dans la formule (I),
composés de formule (II) qui sont mis à réagir avec du diphénylphosphoryle azide en présence de triéthylamine dans du toluène suivi d'addition de tert-butanol pour conduire aux composés de formule (III) : 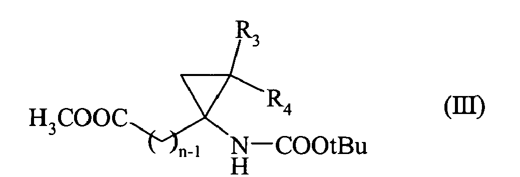 dans laquelle R₃, R₄ et n sont tels que définis précédemment, composés de formule (III) qui sont mis à réagir avec un composé de formule (IV) :

R'₁ - Hal (IV)

dans lequel Hal représente un atome d'halogène et R'₁ représente un groupement choisi parmi alkyle (C₁-C₆) linéaire ou ramifié et arylalkyle (C₁-C₆) linéaire ou ramifié, en présence d'une base dans un solvant anhydre pour conduire aux composés de formule (V) : 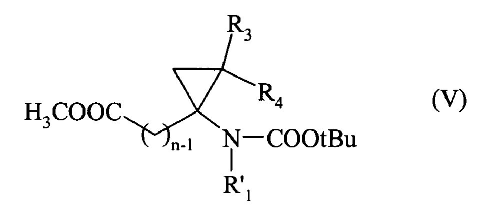 dans laquelle R₃, R₄, n et R'₁ sont tels que définis précédemment,
les composés de formules (III) et (V) formant les composés de formule (VI) : 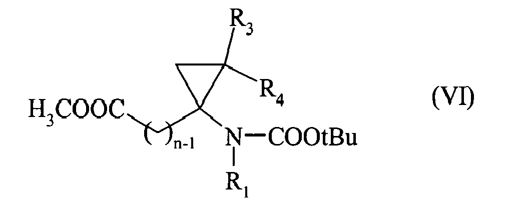 dans laquelle R₃, R₄ et n sont tels que définis précédemment et R₁ est tel que défini dans la formule (I),
composés de formule (VI) qui sont mis en présence d'un agent réducteur pour conduire aux composés de formule (VII) : 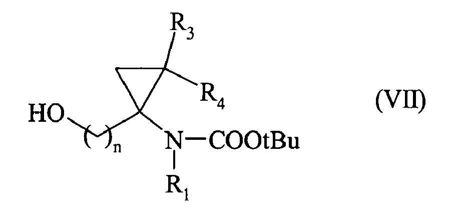 dans laquelle R₃, R₄, n et R₁ sont tels que définis précédemment,
composés de formule (VII) qui sont soumis à l'action d'un agent oxydant classique de la synthèse organique pour conduire aux composés de formule (VIII) : 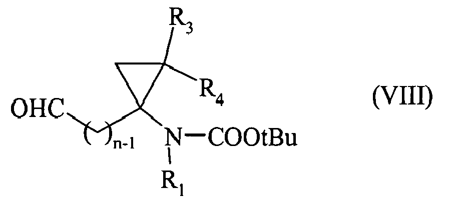 dans laquelle R₃, R₄, n et R₁ sont tels que définis précédemment,
composés de formule (VIII) qui sont mis à réagir avec un composé de formule (IX) : 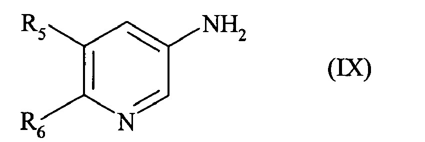 dans laquelle R₅ et R₆ sont tels que définis dans la formule (I), en présence de triacétoxyborohydrure de sodium pour conduire aux composés de formule (X) : 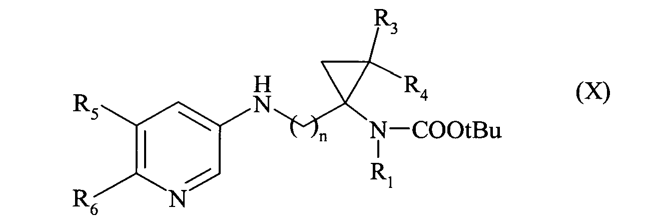 dans laquelle R₃, R₄, R₅, R₆, n et R₁ sont tels que définis précédemment,
composés de formule (X) qui sont :
- soit mis en présence d'un acide dans le dioxane pour conduire aux composés de formule (I/a), cas particulier des composés de formule (I) : 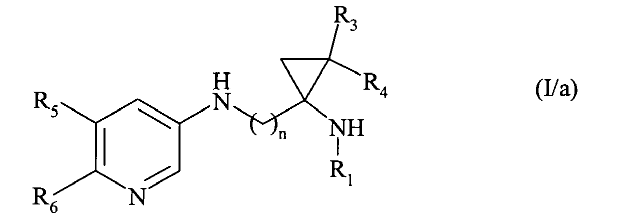
dans laquelle R₁, R₃, R₄, R₅, R₆ et n sont tels que définis précédemment,
composés de formule (I/a) qui sont :
- soit mis en présence de formol et d'acide formique pour conduire aux composés de formule (I/b), cas particulier des composés de formule (I) : 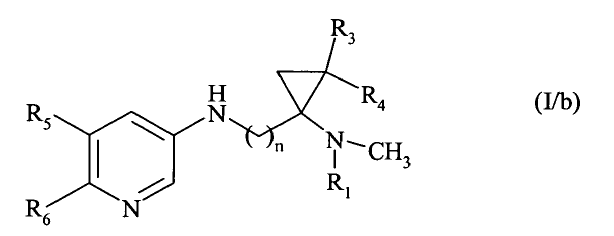
dans laquelle R₁, R₃, R₄, R₅, R₆ et n sont tels que définis précédemment,
- soit mis à réagir avec un composé de formule (XI) :

   R'₂ - Hal (XI)

   dans laquelle Hal est tel que défini précédemment et R'₂ représente un groupement choisi parmi alkyle (C₁-C₆) linéaire ou ramifié et arylalkyle (C₁-C₆) linéaire ou ramifié, en présence d'une base dans un solvant anhydre pour conduire aux composés de formule (I/c), cas particulier des composés de formule (I) : 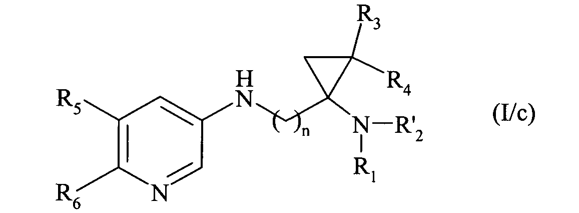
dans laquelle R₁, R₃, R₄, R₅, R₆, n et R'₂ sont tels que définis précédemment,
- soit, lorsque R₁ représente un groupement benzyl, mis en présence d'acide chlorhydrique et de palladium sur charbon, pour conduire aux composés de formule (I/d), cas particulier des composés de formule (I) : 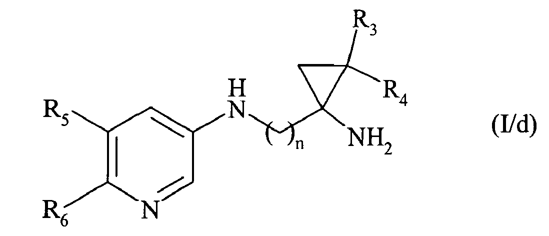
dans laquelle R₃, R₄, R₅, R₆ et n sont tels que définis précédemment,
composés de formule (I/d) qui sont traités par de l'acide formique et une solution aqueuse de formaldéhyde pour conduire aux composés de formule (I/e), cas particulier des composés de formule (I) : 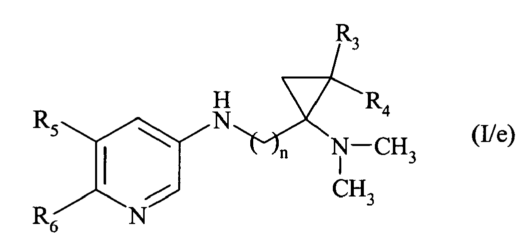 dans laquelle R₃, R₄, R₅, R₆ et n sont tels que définis précédemment,
composés de formules (I/b), (I/c), (I/d) et (I/e) formant les composés de formule (I/f), cas particulier des composés de formule (I) : 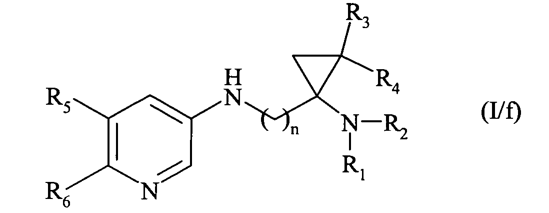 dans laquelle R₁, R₂, R₃, R₄, R₅, R₆ et n sont tels que définis précédemment,
composés de formule (I/f) qui sont mis à réagir avec un composé de formule (XII) :

R'₇ - COOH (XII)

dans laquelle R'₇ représente un groupement choisi parmi alkyle (C₁-C₅) linéaire ou ramifié et arylalkyle (C₁-C₅) linéaire ou ramifié, en présence d'un agent de couplage, suivi d'une réduction de l'amide, pour conduire aux composés de formule (I/g), cas particulier des composés de formule (I) : 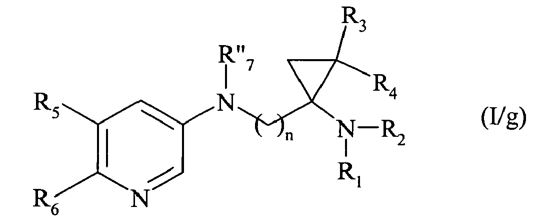 dans laquelle R₁, R₂, R₃, R₄, R₅, R₆ et n sont tels que définis précédemment, et R"₇ représente un groupement choisi parmi alkyle(C₁-C₆) linéaire ou ramifié et arylalkyle (C₁-C₆) linéaire ou ramifié,
- soit mis en présence de carbonyldiimidazole et d'acide formique dans la diméthylformamide pour conduire aux composés de formule (XIII) : 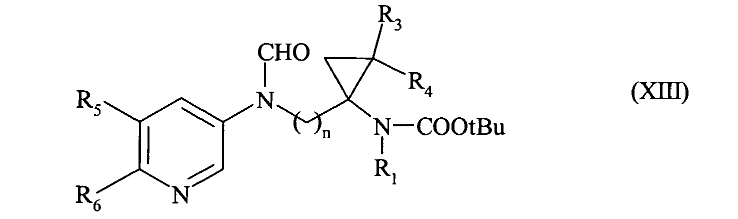
dans laquelle R₁, R₃, R₄, R₅, R₆ et n sont tels que définis précédemment,
composés de formule (XIII) qui sont mis à réagir avec du boraneméthylsulfure complexe dans du tétrahydrofurane pour conduire aux composés de formule (XIV) : 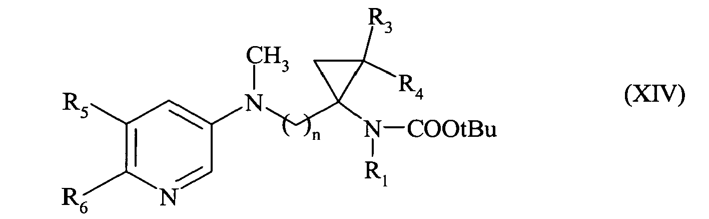 dans laquelle R₁, R₃, R₄, R₅, R₆ et n sont tels que définis précédemment,
composés de formule (XIV) qui sont soumis aux mêmes conditions de réaction que les composés de formule (X), action de l'acide dans le dioxane pour conduire aux composés de formule (I/h), cas particulier des composés de formule (I) : 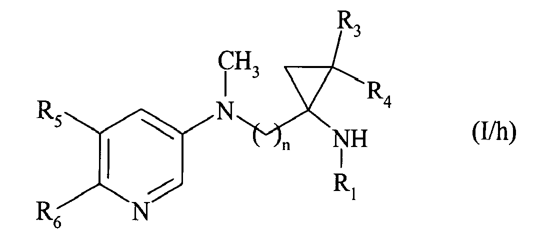 dans laquelle R₁, R₃, R₄, R₅, R₆ et n sont tels que définis précédemment,
composés de formule (I/h) qui sont soumis aux mêmes conditions de réaction que les composés de formule (I/a) lorsque R₁ représente un groupement benzyl, pour conduire aux composés de formule (I/i), cas particuliers des composés de formule (I) : 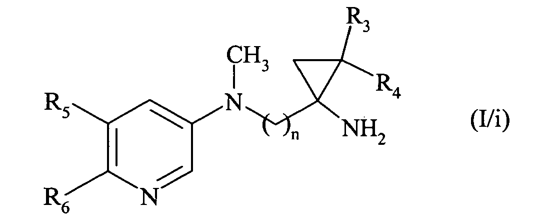 dans laquelle R₃, R₄, R₅, R₆ et n sont tels que définis précédemment, composés de formule (I/i) qui sont soumis aux mêmes conditions de réaction que les composés de formule (I/d), pour conduire aux composés de formule (I/j), cas particuliers des composés de formule (I) : 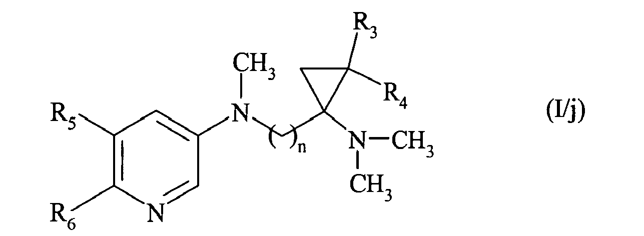 dans laquelle R₃, R₄, R₅, R₆ et n sont tels que définis précédemment,
l'ensemble des composés de formules (I/a) à (I/j) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

Les composés de formules (II), (IV), (IX), (XI) et (XII) sont soit des produits commerciaux, soit obtenus selon des méthodes classiques de la synthèse organique, bien connus de l'homme de l'art.

D'une façon générale, on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon des techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur.

Les composés de l'invention, qui sont présents sous la forme d'un mélange de diastéréoisomères, sont isolés sous forme pure par l'utilisation des techniques classiques de séparation telles que les chromatographies.

Dans certains cas particuliers, le procédé de préparation des composés de l'invention peut conduire à la formation prédominante d'un énantiomère ou d'un diastéréoisomère par rapport à l'autre.

Les composés de la présente invention, de part leurs propriétés pharmacologiques de ligands nicotiniques, et sélectivement du sous-type réceptoriel α4β2, sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisées, la nature et la sévérité de l'affection, et la prise de traitements associés éventuels. La posologie s'échelonne de 1 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utile pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion donné ainsi que la microanalyse élémentaire correspondent à celui du produit salifié.

### PREPARATION I : tert-Butyl 1-(formyl)cyclopropyl(méthyl)carbamate

### Stade 1 : 1-[(tert-Butoxycarbonyl)amino]cyclopropanecarboxylate de méthyle

Une solution de 80 g d'acide 1-(méthoxycarbonyl)cyclopropanecarboxylique, 78 ml de triéthylamine dans 550 ml de toluène, additionnée de 152 g de diphénylphosphoryle azide est chauffée à 80°C. Après arrêt de dégagement gazeux, la température est ramenée à 50°C et 61 g de tert-butanol sont ajoutés. Après 7 heures de réaction à 80°C, le milieu est concentré. Le résidu est repris à l'éther, lavé par une solution saturée en Na₂CO₃ puis par une solution d'acide chlorhydrique 1N, puis par une solution de NaHCO₃. Après séchage, et évaporation de la phase organique, le résidu est repris par 300 ml de cyclohexane, puis concentré à sec. Le résidu obtenu est trituré dans le pentane, filtré puis séché permettant d'isoler le produit attendu.

### Stade 2 : 1-[(tert-Butoxycarbonyl)(méthyl)amino]cyclopropanecarboxylate de méthyle

A une solution refroidie à 5°C de 99,7 g du composé obtenu au stade 1 précédent dans 1,7 1 de diméthylformamide anhydre sont ajoutés par fractions 24,7 g d'hydrure de sodium. Après 15 minutes à 5°C puis 3 heures à température ambiante, 38,2 ml d'iodure de méthyle sont additionnés goutte à goutte. Après 20 heures de réaction, le milieu est évaporé. Le résidu est repris dans l'éther puis traité de façon classique. Une chromatographie sur gel de silice (dichlorométhane) permet d'isoler le produit attendu.

### Stade 3 : tert-Butyl 1-(hydroxyméthyl)cyclopronyl(méthyl)carbamate

A une solution de 23 g du composé obtenu au stade 2 précédent dans 100 ml de tétrahydrofurane est additionnée une solution de 100 ml de borohydrure de lithium 2M dans le tétrahydrofurane. Après 20 heures d'agitation à température ambiante, puis 8 heures au reflux, le milieu réactionnel est refroidi à 0°C, hydrolysé, dilué à l'éther, décanté, séché et concentré. Une chromatographie sur gel de silice du résidu (dichlorométhane/tétrahydrofurane : 95/5) permet d'isoler le produit attendu.

### Stade 4 : tert-Butyl 1-formylcyclopropyl(méthyl)carbamate

A une solution contenant 25,8 g de chlorure d'oxalyle dans 430 ml de dichloromethane, on ajoute, à -60°C, 33,5 g de diméthylsulfoxyde en 20 minutes Après 20 minutes d'agitation à -60°C, on ajoute un mélange contenant 34,3 g du composé du stade 3 précédent dans 100 ml de dichlorométhane en 1 heure à -60°C. Après 30 minutes d'agitation à -60°C, on coule 81 ml de triéthylamine en 20 minutes à -60°C puis on laisse remonter la température à 20°C. On coule 60 ml d'eau, décante et extrait plusieurs fois la phase aqueuse au dichlorométhane. Les phases dichlorométhane jointes sont lavées avec une solution saturée de chlorure de sodium et séchées sur sulfate de sodium puis concentré à sec. Une chromatographie sur gel de silice (dichlorométhane/tétrahydrofurane : 97/3) permet d'obtenir 31,2 g du produit attendu.

### PREPARATION 2 : tert-Butyl benzyl{1-[(pyridin-3-ylamino)méthyl]cyclopropyl} carbamate

### Stade 1 : Méthyl 1-[benzyl(tert-butoxycarbonyl)amino]cyclopropane carboxylate

On introduit 21,5 g du composé du stade 1 de la préparation 1 et 216 ml de diméthylformamide dans un tricol. On ajoute à 20°C, 4,8 g d'hydrure de sodium à 60 % dans l'huile. On agite 2 heures à température ambiante. On coule en 20 minutes 18 ml de bromure de benzyle et on agite 20 heures à température ambiante. On porte 1 heure à 60°C puis on concentre à sec. On reprend le résidu dans l'éther, lave avec une solution à 10 % de carbonate de sodium puis avec une solution à 10 % de chlorure de lithium. On sèche sur sulfate de sodium et concentre à sec. Une chromatographie sur gel de silice (dichlorométhane/cyclohexane : 85/15 puis dichlorométhane pur) permet d'obtenir 21,3 g du produit attendu sous forme de gomme.

### Stade 2 : tert-Butyl benzyl[1-(hydroxyméthyl)cyclopropyl]carbamate

On coule en 20 minutes 70 ml de borohydrure de lithium 2M dans le THF à 20°C dans un mélange de 21,2 g du composé obtenu au stade 1 précédent avec 100 ml de tétrahydrofurane. On agite 20 heures à 20°C puis 1 heure au reflux. On refroidit à 5°C puis on hydrolyse avec précautions par 24 ml d'eau puis 20 ml de solution aqueuse à 10 % de carbonate de sodium. On ajoute 500 ml d'éther, décante et extrait la phase aqueuse à l'éther. Les phases éthérées jointes sont séchées sur sulfate de sodium et concentrées à sec. 19,3 g du produit attendu sont obtenus.
Point de fusion : 68°C

### Stade 3 : tert-Butyl benzyl(1-formylcyclopropyl)carbamate

On coule 10,3 ml de diméthylsulfoxyde en 20 minutes à -60°C dans un mélange de 7,94 ml de chlorure d'oxalyle et de 145 ml de dichlorométhane. On agite 20 minutes à -60°C. On coule 19,3 g du composé obtenu au stade 2 précédent en 1 heure et agite 30 minutes à -60°C. On coule 27,3 ml de triéthylamine et on agite 20 minutes à -60°C. On laisse remonter la température à 20°C et coule 50,6 ml d'eau. Après 10 minutes d'agitation à 20°C, on décante et extrait la phase aqueuse au dichlorométhane. Les phases organiques jointes sont séchées sur sulfate de sodium et concentrées à sec. Une chromatographie sur gel de silice (dichlorométhane) permet d'obtenir 16,45 g du produit attendu.
Point de fusion : 67°C

### Stade 4 : tert-Butyl benzyl{1-[(pyridin-3-ylamino)méthyl]cyclopropyl}carbamate

Dans 2,5 1 de dichlorométhane, on ajoute 38,8 g du produit obtenu au stade 3 précédent, 14,7 g de 3-aminopyridine et 282 ml de tamis moléculaire 3Â. On agite 2 heures à 20°C puis on ajoute 149 g de triacétoxyborohydrure de sodium. On agite 4 jours à 20°C. On filtre et lave le filtrat avec une solution aqueuse à 10 % de carbonate de sodium, sèche sur sulfate de sodium et concentre à sec. Une chromatographie sur gel de silice (dichlorométhane/tétrahydrofurane : 90/10) permet d'obtenir 36,2 g du produit attendu sous forme de gomme.

### EXEMPLE 1 :

### Dichlorhydrate de N-{[1-(méthylamino)cyclopropyl]méthyl}pyridin-3-amine

### Stade 1 : tert-Butyl méthyl{1-[(Pyridin-3-ylamino)méthyl]cyclopropyl}carbamate

On ajoute 15,9 g de triacétoxyborohydrure de sodium sous atmosphère d'azote à 20°C à un mélange contenant 3,0 g de produit de la préparation 1, 300 ml de dichlorométhane, 1,56 g de 3-aminopyridine et 30 ml de tamis moléculaire 3 Å. Le milieu réactionnel est agité 2 jours à 20°C et filtré. Le filtrat est lavé avec une solution à 10 % de carbonate de sodium, séché sur sulfate de sodium et concentré à sec. Une chromatographie sur gel de silice (toluène/éthanol : 95/5) permet d'obtenir 3,0 g du produit attendu.

### Stade 2 : Dichlorhydrate de N{[1-(Méthylamino)cyclopropyl]méthyl}pyridin-3-amine

On coule à 20°C 14 ml d'acide chlorhydrique 4N dans le dioxane dans une solution contenant 1,0 g du produit obtenu au stade 1 précédent dans 50 ml de dioxane. On agite 20 heures à température ambiante. On ajoute de l'éther et essore l'insoluble que l'on dissout dans l'éthanol. On concentre à sec et triture le résidu dans l'éther. On essore les cristaux et sèche sous vide à 30°C. On obtient 0,78 g de produit attendu.
Spectrométrie de masse (ESI) : m/z = 178,1 Th ([M+H]⁺)
Point de fusion : 190-195°C

### EXEMPLE 2 :

### Dichlorhydrate de N-méthyl-N-{[1-(méthylamino)cyclopropyl]méthyl}pyridine-3-amine

### Stade 1 : tert-Butyl (1-{[formyl(pyridin-3-yl)amino]méthyl}cyclopropyl(méthyl) carbamate

On coule une solution de 23,8 g de carbonyldiimidazole et 30 ml de DMF à 5°C dans une solution contenant 6,14 g d'acide formique et 30 ml de DMF. On agite 1 heure à 5°C puis 3 heures à 20°C. On refroidit à 5°C puis on coule un mélange contenant 7,4 g de produit obtenu au stade 1, exemple 1 et 75 ml de DMF. On agite 20 heures à 20°C et on concentre à sec sous 1 torr. On reprend le résidu dans le dichlorométhane, lave avec une solution aqueuse à 10% de carbonate de sodium et sèche sur sulfate de sodium puis on concentre à sec. Une chromatographie sur gel de silice (toluène/éthanol : 95/5) permet d'obtenir 6,2 g de produit attendu.

### Stade 2 : Dichlorhydrate de N-méthyl-N-{[1-(méthylamino)cyclopropyl]méthyl}pyridine-3-amine

On coule à 0°C 1,6 ml de borane méthylsulfure complexe 10M dans un mélange contenant 2,0 g de composé obtenu au stade 1 précédent et 20 ml de tétrahydrofurane. On laisse remonter la température à 40°C puis on porte 3 heures au reflux. On refroidit à 0°C puis on coule 3 ml de méthanol. On agite 1 heure puis on coule lentement du méthanol chlorhydrique jusqu'à pH < 2. On observe un dégagement gazeux. Lorsque le dégagement cesse, on porte au reflux 3 heures. On concentre à sec. On reprend le résidu dans le dichlorométhane puis on lave à la soude 1N. On sèche sur sulfate de sodium et concentre à sec. On chromatographie le résidu sur gel de silice (dichlorométhane/méthanol/ammoniaque 15N : 95/5/0,5). On obtient 0,7 g de base que l'on dissous dans l'éther. On ajoute de l'éther chlorhydrique jusqu'à pH acide. On observe une précipitation. On décante l'éther et triture la gomme avec de l'éther pur. On observe une cristallisation. On essore et sèche à 40°C sous 1 torr. On obtient 0,8 g de produit attendu.
Spectrométrie de masse (ESI) : m/z = 192,1 Th ([M+H]⁺)
Point de fusion : 180-184°C

### EXEMPLE 3 :

### N-{[1-benzylamino)cyclopropyl]méthyl}pyridin-3-amine

On ajoute 50 ml d'acide chlorhydrique 4M dans le dioxane à 20°C à un mélange contenant 7,15 g du composé de la préparation 2, 80 ml de dioxane et 80 ml de méthanol. On agite 3 jours à 20°C. On concentre à sec, ajoute du méthanol, concentre à nouveau et répète 2 fois supplémentaires l'ajout et la distillation du méthanol. On reprend le résidu dans 300 ml de méthanol et ajoute 80 ml de gel de silice. On concentre à sec (empâtage). Une chromatographie sur gel de silice (CH₂Cl₂/Méthanol : 80/20) permet d'obtenir 6,6 g de produit attendu sous forme de gomme.

### EXEMPLE 4 :

### Dichlorhydrate de N-[(1-aminocyclopropyl)méthyl]pyridin-3-amine

On dissous 4,0 g du composé de l'exemple 3 dans 200 ml d'éthanol et on ajoute 1,0 ml d'acide chlorhydrique 11,8 N. On concentre à sec et dissous le résidu dans 200 ml d'éthanol au reflux. Après refroidissement, on ajoute 200 ml de cyclohexène puis sous atmosphère d'azote 1,2 g de palladium sur charbon à 10 %. On porte au reflux 20 heures. On filtre et concentre à sec. On reprend le résidu dans 200 ml d'éthanol et 5 ml d'eau, on concentre à nouveau. Le résidu est dissous dans le méthanol et on ajoute 32 ml de gel de silice (empâtage). On concentre à sec. On chromatographie sur 550 ml de gel de silice (dichlorométhane/méthanol : 80/20). On obtient un composé que l'on reprend dans 12 ml de soude à 35 %. On extrait plusieurs fois à l'éther, sèche les phases éthérées jointes sur sulfate de sodium et concentre à sec. On dissous le résidu dans l'éthanol, ajoute de l'éthanol chlorhydrique jusqu'à pH 1 et concentre à sec. Le résidu est dissous à chaud dans l'isopropanol, refroidit ce qui provoque une cristallisation. On essore et sèche les cristaux à 50°C sous 0,5 torr. On obtient 1,6 g de produit attendu.
Spectrométrie de masse (EI) : m/z = 163,1 Th (M⁺)
Point de fusion : 195-199°C

### EXEMPLE 5 :

### Dichlorhydrate de N-{[1-(diméthylamino)cyclopropyl]méthyl}pyridin-3-amine

On dissous 1,26 g de base du composé de l'exemple 4 dans 25,2 ml d'acide formique et 25,2 ml de formol à 37 %. On porte à 70°C pendant 5 heures. On concentre à sec et reprend dans 20 ml d'eau et concentre à nouveau. On reprend le résidu dans 15 ml de soude à 35 % et extrait à l'éther, sèche les phases éthérées jointes sur sulfate de sodium et concentre à sec. On chromatographie le résidu sur 230 ml de gel de silice (dichlorométhane/méthanol : 95/5) et l'on obtient 1,17 g de résidu que l'on dissous dans 25 ml d'isopropanol. On ajoute 3 ml d'acide chlorhydrique 4M dans le dioxane puis on dilue avec 25 ml d'éther. On essore et sèche les cristaux et l'on obtient 1,25 g de produit attendu.
Spectrométrie de masse (ESI) : m/z = 192,1 Th ([M+H]⁺)
Point de fusion : 208-210°C

### EXEMPLE 6 :

### N-{[1-(Benzylamino)cyclopropyl]méthyl}-N-méthylpyridin-3-amine

### Stade 1 : tert-butylberezyl (1-{(formyl(pyridin-3-yl)amino]méthyl}cyclopropyl)carbamate

Le composé est obtenu selon le procédé du stade 1 de l'exemple 2 en remplaçant le composé du stade 1 de l'exemple 1 par le composé de la préparation 2.

### Stade 2 : N-{[1-(benzylamino)cyclopropyl]méthyl}-N-méthylpyridin-3-amine

Le composé est obtenu selon le procédé du stade 2 de l'exemple 2 en utilisant le composé du stade 1 précédent.

### EXEMPLE 7 :

### Dichlorhydrate de N-{(1-aminocyclopropyl)méthyl]-N-méthylpyridin-3-amine

On ajoute 5,5 ml d'acide chlorhydrique 11,8 N à un mélange contenant 17,7 g du composé de l'exemple 6 avec 900 ml d'éthanol. On tiédit afin d'obtenir une solution. On ajoute 900 ml de cyclohexène puis sous atmosphère d'azote 6 g de palladium sur charbon à 10 %. On porte au reflux pendant 20 heures. On filtre le catalyseur et concentre à sec le filtrat. On reprend le résidu dans 60 ml de soude à 35 % et on extrait abondamment à l'éther. On sèche les phases éthérées jointes sur sulfate de sodium et on concentre à sec. On chromatographie le résidu sur gel de silice (dichlorométhane/méthanol : 93/7). On obtient la base du produit souhaité que l'on salifie par ajout d'acide chlorhydrique en léger excès dans l'éthanol. On dilue avec de l'éther. On essore les cristaux et sèche à 40°C sous 1 torr. On obtient 2,48 g de produit attendu.
Spectrométrie de masse (ESI) : m/z = 178,1 Th ([M+H]⁺)
Point de fusion : 218-222°C

### EXEMPLE 8 :

### Dichlorhydrate de N-{[1-(diméthylamino)cyclopropyl]méthyl}-N-méthylpyridin-3-amine

Le composé est obtenu selon le procédé de l'exemple 5 en remplaçant le composé de l'exemple 4 par le composé de l'exemple 7.
Spectrométrie de masse (ESI) : m/z = 206,2 Th ([M+H]⁺)
Point de fusion : 198-201°C

### EXEMPLE 9 :

### Dichlorhydrate de 6-chloro-N-{[1-(méthylaminocyclopropyl]méthyl}pyridin-3-amine

### Stade 1 : tert-Butyl(1-{[(6-chloropyridin-3-yl)amino]méthyl}cyclopropyl) méthylcarbamate

On ajoute 10 ml d'acide acétique à un mélange contenant 10,8 g du composé de la préparation 1, 100 ml de méthanol et 6,9 g de 5-amino-2-chloropyridine. On agite 30 minutes à température ambiante. On refroidit à 5°C et on ajoute par fractions 4,4 g de cyano borohydrure de sodium. On agite 20 heures à 20°C. On ajoute 10 ml d'eau et concentre à sec. On reprend le résidu dans du dichlorométhane et une solution aqueuse de carbonate de potassium. On décante et extrait plusieurs fois la phase aqueuse au dichlorométhane. Les phases organiques jointes sont séchées sur sulfate de sodium, concentrées à sec. Une chromatographie sur gel de silice (dichlorométhane/tétrahydrofurane : 98/2) permet d'obtenir 9,9 g de produit attendu.

### Stade 2 : Dichlorhydrate de 6-chloro-N-{[1-(méthylaminocycloproyl]méthyl} pyridin-3-amine

Le composé est obtenu selon le procédé du stade 2 de l'exemple 1 en utilisant le composé du stade 1 précédent.
Spectrométrie de masse (ESI) : m/z = 212,1 Th ([M+H]⁺)
Point de fusion : 196-202°C

### EXEMPLE 10 :

### Dichlorhydrate de 6-chloro-N-méthyl-N-{[1-(méthylamino)cyclopropyl)méthyl-pyridin-3-amine

### Stade 1 : tert-Butyl(1-{[(6-chloropyridin-3-yl)(formyl)amino]méthyl}cyclopropyl)méthyl carbamate

Le composé est obtenu selon le procédé du stade 1 de l'exemple 2 en utilisant le composé du stade 1 de l'exemple 9.

### Stade 2 : tert-Butyl(1-{[(6-chloropyridin-3-yl)(méthyl)amino]méthyl}cyclopropyl)méthyl carbamate

On coule à 0°C 2,2 ml de boraneméthylsulfure complexe 10M dans un mélange contenant 3,0 g du produit obtenu au stade 1 précédent et 30 ml de tétrahydrofurane. On cesse de refroidir puis lorsque la température est stabilisée, on porte au reflux pendant 3 heures. Après refroidissement, on coule du méthanol chlorhydrique jusqu'à pH 2. On agite 1 heure à 20°C puis on porte au reflux pendant 1 heure. On concentre à sec et reprend le résidu par un mélange de dichlorométhane et de soude 4N. On décante et extrait la phase aqueuse au dichlorométhane. Les phases organiques jointes sont séchées sur sulfate de sodium et concentrées à sec. Le résidu est chromatographié sur 200 g de gel de silice (dichlorométhane/tétrahydrofurane : 95/5). On obtient 1,6 g de produit attendu.

### Stade 3 : Dichlorhydrate de 6-chloro-N-méthyl-N-{[1-méthylamino)cyclopropyl] méthylpyridin-3-amine

Le produit est obtenu selon le procédé du stade 2 de l'exemple 1 en utilisant le produit obtenu au stade 2 précédent.
Spectrométrie de masse (ESI) : m/z = 226,1 Th ([M+H]⁺)
Point de fusion : 133-136°C

### EXEMPLE 11 :

### Fumarate de 6-bromo-N-{[1-(méthylaminocyclopropyl]méthyl}pyridin-3-amine

### Stade 1 : tert-Butyl(1-{[(6-bromonyridin-3-yl)amino]méthyl}cycloyropyl) méthylcarbamate

Le produit est obtenu selon le procédé du stade 1 de l'exemple 9 en utilisant la 5-amino-2-bromopyridine à la place de la 5-amino-2-chloropyridine.

### Stade 2 : Fumarate de 6-bromo-N-{[1-(méthylamino)cyclopropyl]méthyl}pyridin-3-amine

On ajoute 3,5 ml d'acide trifluoroacétique dans un mélange contenant 2 g du produit obtenu au stade 1 précédent et 20 ml du dichlorométhane. On agite 20 heures à 20°C. On ajoute une solution aqueuse à 10 % de carbonate de sodium jusqu'à ce que le pH soit > 9. On ajoute du dichlorométhane. On décante, sèche la phase organique sur sulfate de sodium et concentre à sec. On reprend le résidu dans 5 ml d'éthanol. On ajoute 0,6 g d'acide fumarique dissous dans 10 ml d'éthanol. On observe une cristallisation. On essore, lave à l'éthanol puis à l'éther et on sèche à 50°C sous 1 torr. On obtient 1,6 g de produit attendu.
Spectrométrie de masse (ESI) : m/z = 256,0 Th ([M+H]⁺)
Point de fusion : 165-169°C

### EXEMPLE 12 :

### Fumarate de 6-bromo-N-méthyl-N-{[1-(méthylamino)cyclopropyl)méthyl}pyridin-3-amine

### Stade 1 : tert-Butyl(1-{[(6-bromopyridin-3-yl)(formyl)amino]méthyl}cyclopropyl) méthylcarbamate

Le produit est obtenu selon le procédé du stade 1 de l'exemple 2 en utilisant le composé du stade 1 de l'exemple 11.

### Stade 2 : tert-Butyl(1-{[(6-bromopyridin-3-yl)(méthyl)amino]méthyl}cyclopropyl) méthylcarbamate

Le produit est obtenu selon le procédé du stade 2 de l'exemple 10 en utilisant le produit du stade 1 précédent.

### Stade 3 : Fumarate de 6-bromo-N-méthyl-N-{[1-(méthylamino)cyclopropyl]méthyl} pyridine-3-amine

Le produit est obtenu selon le procédé du stade 2 de l'exemple 11 en utilisant le produit obtenu au stade 2 précédent.
Spectrométrie de masse (ESI) : m/z = 270,1 Th ([M+H]⁺)
Point de fusion : 146-150°C

### EXEMPLE 13 :

### Dichlorhydrate de 6-méthyl-N-{[1-(méthylamino)cyclopropyl]méthyl}pyridin-3-amine

### Stade 1 : tert-Butyl méthyl(1-{[(6-méthylpyridin-3-yl)amino]méthyl}cyclopropyl) carbamate

Le composé est obtenu selon le procédé du stade 1 de l'exemple 9 en utilisant la 5-amino-2-méthylpyridine à la place de la 5-amino-2-chloropyridine.

### Stade 2 : Dichlorhydrate de 6-méthyl-N-{[1-(méthylamino)cyclopropyl]méthyl}pyridin-3-amine

Le composé est obtenu selon le procédé du stade 2 de l'exemple 9 en utilisant le produit du stade 1 précédent.
Spectrométrie de masse (ESI) : m/z = 192,2 Th ([M+H]⁺)
Point de fusion : 230-232°C

### EXEMPLE 14 :

### Dichlorohydrate de N,6-diméthyl-N-{[1-(méthylamino)cyclopropyl]méthyl}pyridin-3-amine

### Stade 1 : tert-Butyl(1-{[formyl(6-méthylpyridin-3-ylamino]méthyl}cyclopropyl) méthylcarbamate

Le composé est obtenu selon le procédé du stade 1 de l'exemple 2 en utilisant le composé du stade 1 de l'exemple 13.

### Stade 2 : tert-Butylméthyl(1-{[méthyl(6-méthylpyridin-3-yl)amino]méthyl}cyclopropyl) carbamate

Le composé est obtenu selon le procédé du stade 2 de l'exemple 2 en utilisant le produit du stade 1 précédent.

### Stade 3 : Dichlorhydrate de N,6-diméthyl-N-{[1-(méthylamino)cyclopropyl]méthyl} pyridin-3-amine

Le composé est obtenu selon le procédé du stade 2 de l'exemple 1 en utilisant le produit obtenu au stade 2 précédent.
Spectrométrie de masse (ESI) : m/z = 206,2 Th ([M+H]⁺)
Point de fusion : 112-115°C

### EXEMPLE 15 :

### Dichlorhydrate de 6-chloro-N-{[1-(diméthylamino)cyclopropyl]méthyl}pyridin-3-amine

Le composé est obtenu selon le procédé de l'exemple 5 en remplaçant le composé de l'exemple 4 par le composé de l'exemple 9.
Spectrométrie de masse (EI) : m/z 225.1 Th (M⁺)
Point de fusion : 158-160 °C

### EXEMPLE 16 :

### Dichlorhydrate de 6-chloro-N-{[1-(diméthylamino)cyclopropyl]méthyl}-N-méthylpyridin-3-amine

### Stade 1 : (6-chloropyridin-3-yl)({1-[formyl(méthyl)amino]cyclopropyl}méthyl) formamide

On ajoute en 15 minutes 1,85 ml d'acide formique à 3,7 ml d'anhydride acétique à 5°C, puis on chauffe ce mélange 2 heures à 55°C. Après retour à température ambiante, on ajoute 7,4 ml de tétrahydrofuranne puis le mélange réactionnel est refroidi à -20°C. On coule en 30 minutes une solution de 1,57 g de la base du composé de l'exemple 9 dans 18,5 ml de tétrahydrofuranne. On maintient une heure à -20°C puis 20 heures à 0°C. On concentre, on reprend le résidu par une solution aqueuse à 10% de carbonate de sodium et on extrait au dichlorométhane. On sèche le dichlorométhane sur sulfate de sodium et on concentre à sec. Une chromatographie sur gel de silice (dichlorométhane/méthanol : 97,5/2,5) permet d'obtenir 1,73 g du produit attendu.

### Stade 2 : Dichlorhydrate 6-chloro-N-{[1-(diméthylamino)cyclopropyl]méthyl}-N-méthylpyridin-3-amine

On dissous 1,73 g du composé obtenu au stade 1 précédent dans 48 ml de tétrahydrofuranne puis on ajoute 3,6 ml de complexe borane-méthylsulfure 10M. On agite 20 heures à 20°C puis on porte 3 heures au reflux. On refroidit à 5°C et on coule 6,5 ml de méthanol, on agite une heure et on concentre à sec. On reprend au dichlorométhane, on lave avec une solution aqueuse à 10 % d'hydrogénocarbonate de sodium. La phase organique est séchée sur sulfate de sodium, concentrée et une chromatographie du résidu sur gel de silice (dichlorométhane/méthanol : 97,5/2,5) permet d'obtenir la base du produit attendu. 0,7g de cette base sont dissous dans 5 ml d'isopropanol et on ajoute une solution d'acide chlorhydrique dans l'éther pour obtenir, après filtration d'un précipité et séchage, 0,85 g du composé attendu.
Spectrométrie de masse (ESI) : m/z 240.1 Th ([M+H]⁺)
Point de Fusion : 163-166 °C

### EXEMPLE 17 :

### Dichlorhydrate de N-benzyl-6-chloro-N-{[1-(méthylamino)cyclopropyl]méthyl} pyridin-3-amine

### Stade 1 : tert-butyl (1-{[benzoyl(6-chloropyridin-3-yl)amino]méthyl}cyclopropyl) méthylcarbamate

On dissous 10,17 g du composé obtenu au stade 1 de l'exemple 9 dans 200ml de tétrahydrofuranne, on ajoute 4,88 ml de triéthylamine, puis on refroidit à 5°C et on coule, en 30 minutes, 5,05g de chlorure de benzoyle. On agite une heure à 5°C, puis une heure à température ambiante, puis on porte 2 heures au reflux. On concentre à sec, on reprend le résidu avec du dichlorométhane et on lave avec une solution aqueuse à 50% de carbonate de potassium. La phase organique est séchée sur sulfate de sodium, concentrée et une chromatographie du résidu sur gel de silice (dichlorométhane/tétrahydrofuranne : 96/4) permet d'obtenir 13,9g de produit attendu.

### Stade 2 : tert-butyl (1-{[benzyl(6-chloropyridin-3-yl)amino]méthyl}cyclopropyl) méthylcarbamate

On dissous 13,8 g du composé obtenu au stade 1 précédent dans 120 ml de tétrahydrofuranne, on refroidit à 5 °C puis on ajoute 8,3 ml de complexe borane― méthylsulfure 10M. Après retour à température ambiante on porte 3 heures au reflux. On refroidit à 5°C et on coule 15,1 ml de méthanol, on agite une heure puis on acidifie à pH 3 avec une solution d'acide chlorhydrique dans le méthanol. On porte une heure au reflux puis on concentre à sec. On reprend au dichlorométhane, on lave avec une solution aqueuse à 10 % de carbonate de sodium. La phase organique est séchée sur sulfate de sodium, concentrée et une chromatographie du résidu sur gel de silice (dichlorométhane à dichlorométhane/butanone : 90/10) permet d'obtenir 6,7g du composé attendu.

### Stade 3 : Dichlorhydrate de N-benzyl-6-chloro-N-{[1-(méthylamino)cyclopropyl]méthyl} pyridin-3-amine

Le composé est obtenu selon le procédé du stade 2 de l'exemple 1 en remplaçant le composé du stade 1 de l'exemple 1 par le composé du stade 2 précédent.
Spectrométrie de masse (ESI) : m/z 302.1 Th ([M+H]⁺)
Point de Fusion : 156-157 °C

### EXEMPLE 18 :

### Chlorhydrate de N-benzyl-6-chloro-N-{[1-(diméthylamino)cyclopropyl]méthyl} pyridin-3-amine

On dissous 3,1g de la base du composé de l'exemple 17 dans 60 ml d'acide formique. On ajoute 60 ml d'une solution aqueuse à 37% de formaldéhyde et on porte 4h à 70°C. On concentre à sec, on reprend le résidu avec une solution aqueuse à 50% de carbonate de potassium et on extrait au dichlorométhane. On sèche la phase organique sur sulfate de sodium, on concentre et on chromatographie le résidu sur gel de silice (dichlorométhane/acétone : 96/4). On isole une fraction de 0,48 g de base du composé attendu qui, après dissolution dans 5 ml d'éthanol et ajout d'une solution d'acide chlorhydrique dans l'éther permet d'obtenir, après filtration d'un précipité et séchage, 0,4 g du composé attendu.
Spectrométrie de masse (ESI) : m/z 316,16 Th ([M+H]⁺)
Point de Fusion : 190-192°C

### ETUDES PHARMACOLOGIQUES DES COMPOSES DE L'INVENTION

### EXEMPLE A :

### Déplacement de la fixation de l'[¹²⁵I]-α-bungarotoxine sur les récepteurs nicotiniques de l'organe électrique de torpille

Cette étude, réalisée selon la méthode décrite dans J. Pharmacol. Exp. Ther., 1994, 271 ; 624-631, a pour objectif d'évaluer l'affinité des composés de la présente invention sur les récepteurs nicotiniques de « type musculaire ».
Des membranes (1-5 µg/ml) d'organe électrique de torpille sont incubées (1h, 22°C) en présence d'une gamme de concentration (0,01-10 µM) de chaque composé de l'invention (dilution à partir d'une solution mère à 10 mM dans du DMSO) en présence d'[¹²⁵I]-α-bungarotoxine (A.S. : 7,4 TBq/mmol : 0,2 nM) dans du tampon Krebs (Tris-HCl 50 mM, KCl 5 mM, MgCl₂ 1 mM, CaCl₂ 2 mM, NaCl 100 mM, pH 7.4) avec 0,01 % de BSA, volume final de 500 µl. La liaison non-spécifique est déterminée par l'incubation de membranes en présence d'α-bungarotoxine (1 µM).
Les résultats indiquent que tous les composés de la présente invention ne possèdent aucune affinité significative vis-à-vis des récepteurs nicotiniques de « type musculaire », jusqu'à la concentration de 10 µM (Kᵢ > 10⁻⁵M).

### EXEMPLE B :

### Déplacement de la fixation d'[³H]-épibatidine sur les récepteurs nicotiniques de cellules IMR32

Cette étude, réalisée selon la technique décrite dans Molec. Pharmacol., 1995, 48 ; 280-287, a pour but de déterminer l'affinité des composés de la présente invention sur les récepteurs nicotiniques de « type ganglionnaire » (American Soc. Neuroscience, 2000, 26, 138).
Des membranes (250 µg/ml) de cellules neuroblastome IMR-32 sont incubées (2h, 20°C) en présence d'une gamme de concentration (0,01-10 µM) de chaque composé de l'invention (dilution à partir d'une solution mère à 10 mM dans du DMSO) et d'(±)-[³H]-épibatidine (A.S.: 2464 GBq/mmol: 1,5 nM) dans du tampon phosphate (NaH₂PO₄ 20 mM, pH 7,4), volume final de 250 µl. La liaison non-spécifique est déterminée par l'incubation de membranes en présence de 300 µM de (-)nicotine.
Les résultats montrent que tous les composés de la présente invention n'ont aucune affinité significative sur les récepteurs nicotiniques de « type ganglionnaire », jusqu'à la concentration de 10 µM (Kᵢ > 10⁻⁵M).

### EXEMPLE C :

### Déplacement de la fixation d'[³H]-oxotrémorine-M sur les récepteurs muscariniques de cerveau de rat

Cette étude, réalisée selon la méthode décrite dans Naumyn-Schmiederberg's Arch. Pharmacol., 2001, 363, 429-438, a pour objectif de déterminer l'affinité des composés de la présente invention sur les récepteurs muscariniques.
Des membranes (250 µg/ml) de cerveau de rat sont incubées (2h, 20°C) en présence d'une gamme de concentration (0,01-10 µM) de chaque composé de l'invention (dilution à partir d'une solution mère à 10 mM dans du DMSO) et d'[³H]-oxotrémorine-M (A.S. : 3174 GBq/mmol : 2 nM) dans du tampon phosphate (NaH₂PO₄ 20 mM, pH 7,4), volume final de 250 µl. La liaison spécifique est déterminée par l'incubation des membranes en présence d'atropine (1 µM). L'affinité des composés de la présente invention vis-à-vis des récepteurs muscariniques est caractérisée par la détermination du Kᵢ.
Les résultats démontrent que tous les composés de la présente invention, jusqu'à la concentration de 10 µM, sont dépourvus d'affinité vis-à-vis des récepteurs muscariniques (Kᵢ > 10⁻⁵M).

### EXEMPLE D :

### Déplacement de la fixation de l'[¹²⁵I]-α-bungarotoxine sur les récepteurs nicotiniques de « type α7 » de cerveau de rat

Cette étude, réalisée selon la méthode décrite dans Molec. Pharmacol., 1986, 30 ; 427-436, a pour objectif de déterminer l'affinité des composés de la présente invention vis-à-vis des récepteurs centraux nicotiniques de type α7.
Des membranes (1000 µg/ml) de cerveau de rat sont incubées (5h, 37°C) en présence d'une gamme de concentration (0,01-10 µM) de chaque composé de la présente invention (dilution à partir d'une solution mère à 10 mM dans du DMSO) et d'[¹²⁵I]-α-bungarotoxine (A.S. : 7,4 TBq/mmol : 1 nM) dans du tampon Krebs (Tris-HCl 50 mM, KCl 5 mM, MgCl₂ 1 mM, CaCl₂ 2 mM, NaCl 100 mM, pH 7,4) avec 0,05 % de BSA, volume final de 500 µl. La liaison non-spécifique est déterminée par l'incubation de membranes en présence d'α-bungarotoxine (1 µM).

L'affinité des composés de la présente invention vis-à-vis des récepteurs nicotiniques de type α7 est caractérisée par la détermination du Kᵢ.
Les résultats indiquent que la plupart des composés de la présente invention, jusqu'à la concentration de 10 µM, sont dépourvus d'affinité vis-à-vis des récepteurs nicotiniques centraux de type α7. Certains composés de l'invention présentent un Kᵢ de l'ordre de 10 µM comme l'exemple 2 qui possède un Kᵢ égal à 8.3 x 10⁻⁶M.

### EXEMPLE E :

### Déplacement de la fixadon de [³H]-cytisine sur les récepteurs nicotiniques de « type α4β2 » de cerveau de rat

Cette étude, réalisée selon la technique décrite dans Molec. Pharmacol., 1990, 39 ; 9-12, a pour objectif de déterminer l'affinité des composés de la présente invention vis-à-vis des récepteurs nicotiniques centraux de type α4β2.
Des membranes (250 µg/ml) de cerveau de rat sont incubées (2h, 20°C) en présence d'une gamme de concentration (0,01-10 µM) de chaque composé de la présente invention (dilution à partir d'une solution mère à 10 mM dans du DMSO) et de [³H]-cytisine (A.S. : 1184 GBq/mmol : 2 nM) dans du tampon phosphate (NaH₂PO₄ 20 mM, pH 7,4), volume final de 250µl. La liaison non-spécifique est déterminée par l'incubation de membranes en présence de 10 µM de (-)nicotine. L'affinité des composés de la présente invention vis-à-vis des récepteurs nicotiniques centraux de type α4β2 est caractérisée par la détermination du Kᵢ.
Les résultats obtenus montrent que les composés de la présente invention présentent une forte affinité vis-à-vis des récepteurs nicotiniques centraux de type α4β2. Ainsi les exemples 1, 2 et 10 présentent des Kᵢ de 3.4 x 10⁻⁸M, 1.5 x 10⁻⁸M et 1.6 x 10⁻⁸M respectivement.

Ces résultats, ainsi que ceux obtenus dans les exemples A à D indiquent que les composés de la présente invention sont de puissants ligands nicotiniques centraux spécifiques des récepteurs de type α4β2.

### EXEMPLE F :

### Mesure in vivo de la libération d'acétylcholine par microdialyse intra-corticale chez le rat Wistar vigile

L'administration systémique de nicotine et d'agonistes nicotiniques induit une augmentation *in vivo* d'acétylcholine dans diverses régions cérébrales (Neurochem. Res., 1996, 21, 1181-1186 ; Eur. J. Pharmacol., 1998, 351, 181-188 ; Br. J. Pharmacol., 1999, 127, 1486-1494). Une sonde de microdialyse est implantée au niveau du cortex préfrontal médian de rats mâles Wistar. Six ou sept jours après l'implantation des sondes, celles-ci sont perfusées par du Ringer (NaCl 147 mM, KCl 2.7 mM, CaCl₂ 1.2 mM, MgCl₂ 1 mM, néostigmine 20 nM) à un débit de 1 µl/min chez l'animal libre de se mouvoir. Après 2 heures de stabulation, le produit étudié est administré par voie intrapéritonéale. Un groupe d'animaux témoins reçoit le solvant du produit. Puis, les dialysats (30 µl) sont collectés toutes les 30 minutes pendant 4h afin de mesurer les concentrations extra-synaptiques corticales d'acétylcholine par HPLC en détection ampérométrique. Les résultats sont exprimés en pg d'acétylcholine/dialysat et les comparaisons inter-groupes sont effectuées par une analyse de variance à 2 facteurs (traitement x temps) avec mesures répétées sur le temps.

Les résultats obtenus montrent que les composés de la présente invention augmentent la libération corticale *in vivo* d'acétylcholine de manière dose-dépendante pour des doses actives allant de 0,3 à 3 mg/kg IP et indiquent le caractère agoniste α4β2. Ainsi les exemples 1 et 2, une heure après leur administration à la dose de 3 mg/kg IP, induisent une augmentation de +70 % et + 80 % respectivement, de la libération d'acétylcholine dans le cortex préfrontal chez le rat Wistar vigile.

### EXEMPLE G :

### Torsions abdominales induites à la phényl-p-benzoquinone (PBQ) chez la souris NMRI

L'administration intrapéritonéale d'une solution alcoolique de PBQ provoque des crampes abdominales chez la Souris (Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (Brit. J. Pharmacol. Chem., 1968, 32, 295-310). A t=0 minute, les animaux sont pesés et le produit étudié est administré par voie IP. Un groupe d'animaux témoins reçoit le solvant du produit. A t=30 minutes, une solution alcoolique de PBQ (0.2 %) est administrée par voie IP sous un volume de 0,25 ml/souris. Immédiatement après l'administration de la PBQ, les animaux sont placés dans des cylindres en plexiglass (L = 19,5 cm ; D.I. = 5 cm). De t=35 minutes à t=45 minutes, la réaction des animaux est observée et l'expérimentateur note le nombre total de crampes abdominales par animal. Les résultats sont exprimés par le pourcentage d'inhibition du nombre de crampes abdominales mesuré chez les animaux témoins à la dose active du composé étudié.

Les résultats obtenus montrent une inhibition de l'ordre de -80 %, pour des doses actives de 10 mg/kg IP. Ceci montre que les composés de l'invention sont pourvus de propriétés antalgiques. Ainsi les exemples 1, 2, 9 et 10, administrés à la dose de 10 mg/kg IP, diminuent le nombre de crampes abdominales provoquées par l'administration de PBQ chez la souris de -50 %, -76 %, -52 % et -69 % respectivement.

### EXEMPLE H :

### Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368 ; Psychophamacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des 2 rencontres.

Les résultats obtenus montrent une différence (T2-T1) comprise entre -19 et -36 s pour des doses allant de 1 à 3 mg/kg IP. Ceci montre que les composés de l'invention augmentent la mémorisation de façon très importante, et à faible dose. Ainsi, les exemples 1, 2 et 4, à la dose de 3 mg/kg IP, induisent une différence (T2-T1) de 36, 29 et 19s, respectivement.

### EXEMPLE I :

### Reconnaissance d'objet chez le Rat Wistar

Le test de la reconnaissance d'objet chez le rat Wistar (Behav. Brain Res., 1988, 31, 47-59) est basé sur l'activité exploratoire spontanée de l'animal et possède les caractéristiques de la mémoire épisodique chez l'homme. Sensible au vieillissement (Eur. J. Pharmacol., 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explore de façon préférentielle l'objet nouveau, ce qui signe le souvenir de l'objet déjà présenté.
Les résultats obtenus montrent une différence Delta de l'ordre de 9 s, pour des doses allant de 0,01 à 0,3 mg/kg PO, ce qui montre que les composés de l'invention augmentent la mémorisation de façon importante, et à très faible dose. Ainsi, administrés à la dose de 0,3 mg/kg PO, les exemples 1 et 2 provoquent une différence Delta de 9 et 4 s, respectivement.

### EXEMPLE J :

### Compositions pharmaceutiques pour 1000 comprimés dosés à 10 mg

| | |
|---|---|
| Composé de l'exemple 1 | 10 g |
| Hydroxypropylméthylcellulose | 10 g |
| Amidon de blé | 15 g |
| Lactose | 90 g |
| Stéarate de magnésium | 2 g |

## Revendications

1. Composé de formule (I) : 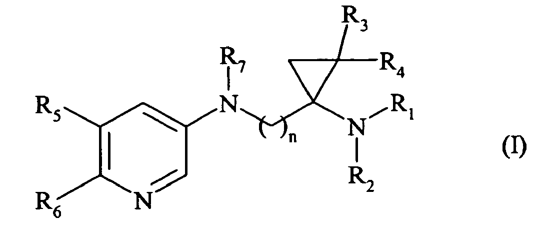 dans laquelle :
n représente un nombre entier compris entre 1 et 6 inclus,
R₁ et R₂, identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
R₃ et R₄, identiques ou différents, indépendamment l'un de l'autre, représentent un atome d'hydrogène, ou un groupement alkyle (C₁-C₆) linéaire ou ramifié,
R₅ et R₆, identiques ou différents, indépendamment l'un de l'autre, représentent un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, ou amino éventuellement substitué par un ou deux groupements alkyle (C₁-C₆) linéaire ou ramifié,
R₇ représente un atome d'hydrogène, ou un groupement alkyle (C₁-C₆) linéaire ou ramifié ou arylalkyle (C₁-C₆) linéaire ou ramifié,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié, leurs énantiomères, diastéréoisomères, ainsi que leurs sels d'addition à un acide ou à une base pharmaceutiquement acceptable.

2. Composés de formule (I) selon la revendication 1, **caractérisés en ce que** n est un entier prenant la valeur 1, leurs énantiomères, diastéréoisomères, ainsi que leurs sels d'addition à un acide ou à une base pharmaceutiquement acceptable.

3. Composés de formule (I) selon la revendication 1, **caractérisés en ce que** R₁ et R₂, identiques ou différents, indépendamment l'un de l'autre, représentent un atome d'hydrogène et un groupement alkyle (C₁-C₆) linéaire ou ramifié, leurs énantiomères, diastéréoisomères, ainsi que leurs sels d'addition à un acide ou à une base pharmaceutiquement acceptable.

4. Composés de formule (I) selon la revendication 1, **caractérisés en ce que** R₃ et R₄ représentent un atome d'hydrogène, leurs énantiomères, diastéréoisomères, ainsi que leurs sels d'addition à un acide ou à une base pharmaceutiquement acceptable.

5. Composés de formule (I) selon la revendication 1, **caractérisés en ce que** R₅ et R₆, identiques ou différents, indépendamment l'un de l'autre, représentent un atome d'hydrogène et un groupement alkyle (C₁-C₆) linéaire ou ramifié, leurs énantiomères, diastéréoisomères, ainsi que leurs sels d'addition à un acide ou à une base pharmaceutiquement acceptable.

6. Composés de formule (I) selon la revendication 1, **caractérisés en ce que** R₇ représente un atome d'hydrogène et un groupement alkyle (C₁-C₆) linéaire ou ramifié, leurs énantiomères, diastéréoisomères, ainsi que leurs sels d'addition à un acide ou à une base pharmaceutiquement acceptable.

7. Composés de formule (I) selon la revendication 1 qui sont le :
*N-*{[1-(méthylamino)cyclopropyl]méthyl}pyridin-3-amine
*N*-méthyl-*N*-{[1-(méthylamino)cylopropyl]méthyl}pyridin-3-amine
*N*-[(1-aminocyclopropyl)méthyl] pyridin-3-amine
*N*-{[1-(diméthylamino)cyclopropyl]méthyl}pyridin-3-amine
*N*-[(1-aminocyclopropyl)méthyl]-*N*-méthylpyridin-3-amine
*N*-{[1-(diméthylamino)cyclopropyl]méthyl}-*N*-méthylpyridin-3-amine
6-chloro-*N*-{[1-(méthylamino)cyclopropyl]méthyl}pyridin-3-amine
6-chloro-*N*-méthyl-*N*-{[1-(méthylamino)cyclopropyl]méthyl}pyridin-3-amine
6-bromo-*N*-{[1-(méthylamino)cyclopropyl]méthyl}pyridin-3-amine
6-bromo-*N*-méthyl-*N*-{[1-(méthylamino)cyclopropyl]méthyl}pyridin-3-amine
6-méthyl-*N*-{[1-(méthylamino)cylopropyl]méthyl}pyridin-3-amine
*N*,6-diméthyl-*N*-{[1-(méthylamino)cyclopropyl]méthyl}pyridin-3-amine,
leurs énantiomères, diastéréoisomères, ainsi que leurs sels d'addition à un acide ou à une base pharmaceutiquement acceptable.

8. Procédé de préparation des composés de formule (I), selon la revendication 1, **caractérisé en ce qu'**on utilise comme produit de départ un composé de formule (II) : 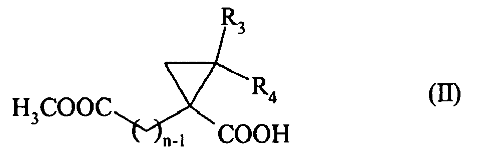 dans lequel R₃, R₄ et n sont tels que définis dans la formule (I),
composés de formule (II) qui sont mis à réagir avec du diphénylphosphoryle azide en présence de triéthylamine dans du toluène suivi d'addition de tert-butanol pour conduire aux composés de formule (III) : 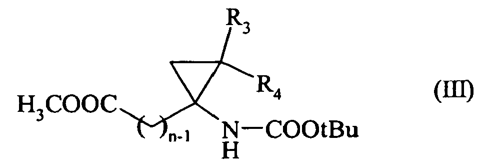 dans laquelle R₃, R₄ et n sont tels que définis précédemment, composés de formule (III) qui sont mis à réagir avec un composé de formule (IV) :
R'₁ - Hal (IV)
dans lequel Hal représente un atome d'halogène et R'₁ représente un groupement choisi parmi alkyle (C₁-C₆) linéaire ou ramifié et arylalkyle (C₁-C₆) linéaire ou ramifié, en présence d'une base dans un solvant anhydre pour conduire aux composés de formule (V) : 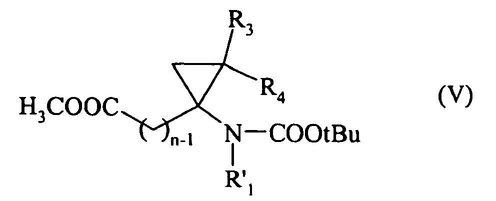 dans laquelle R₃, R₄, n et R'₁ sont tels que définis précédemment,
les composés de formules (III) et (V) formant les composés de formule (VI) : 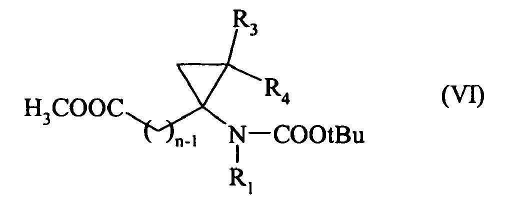 dans laquelle R₃, R₄ et n sont tels que définis précédemment et R₁ est tel que défini dans la formule (I),
composés de formule (VI) qui sont mis en présence d'un agent réducteur pour conduire aux composés de formule (VII) : 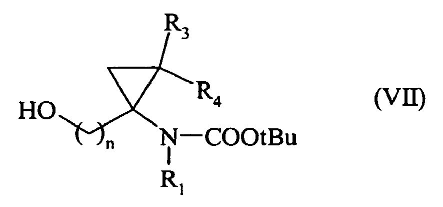 dans laquelle R₃, R₄, n et R₁ sont tels que définis précédemment,
composés de formule (VII) qui sont soumis à l'action d'un agent oxydant classique de la synthèse organique pour conduire aux composés de formule (VIII) : 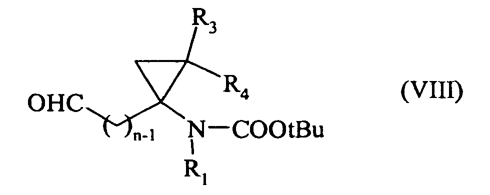 dans laquelle R₃, R₄, n et R₁ sont tels que définis précédemment,
composés de formule (VIII) qui sont mis à réagir avec un composé de formule (IX) : 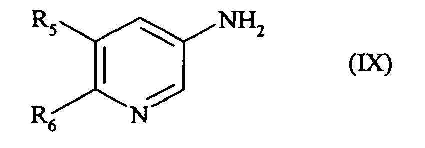 dans laquelle R₅ et R₆ sont tels que définis dans la formule (I), en présence de triacétoxyborohydrure de sodium pour conduire aux composés de formule (X) : 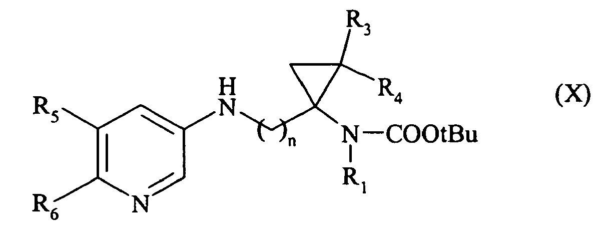 dans laquelle R₃, R₄, R₅, R₆, n et R₁ sont tels que définis précédemment,
composés de formule (X) qui sont :
- soit mis en présence d'un acide dans le dioxane pour conduire aux composés de formule (I/a), cas particulier des composés de formule (I) : 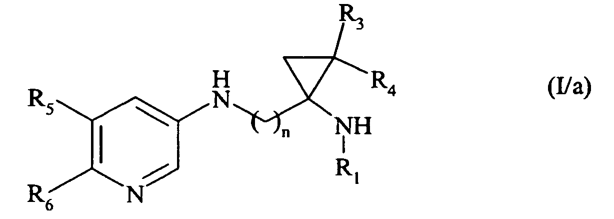
dans laquelle R₁, R₃, R₄, R₅, R₆ et n sont tels que définis précédemment,
composés de formule (I/a) qui sont :
- soit mis en présence de formol et d'acide formique pour conduire aux composés de formule (I/b), cas particulier des composés de formule (I) : 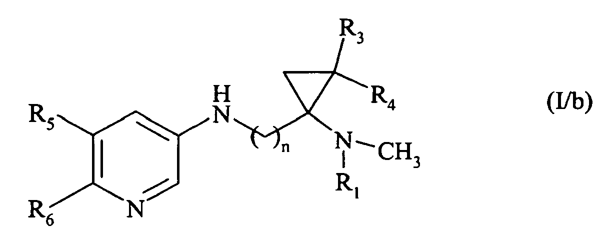
dans laquelle R₁, R₃, R₄, R₅, R₆ et n sont tels que définis précédemment,
- soit mis à réagir avec un composé de formule (XI) :
R'₂ - Hal (XI)
dans laquelle Hal est tel que défini précédemment et R'₂ représente un groupement choisi parmi alkyle (C₁-C₆) linéaire ou ramifié et arylalkyle (C₁-C₆) linéaire ou ramifié, en présence d'une base dans un solvant anhydre pour conduire aux composés de formule (I/c), cas particulier des composés de formule (I) : 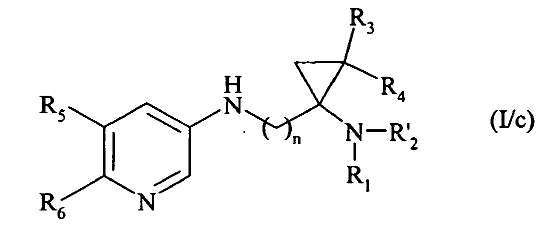 dans laquelle R₁, R₃, R₄, R₅, R₆, n et R'₂ sont tels que définis précédemment,
- soit, lorsque R₁ représente un groupement benzyl, mis en présence d'acide chlorhydrique et de palladium sur charbon, pour conduire aux composés de formule (I/d), cas particulier des composés de formule (I) : 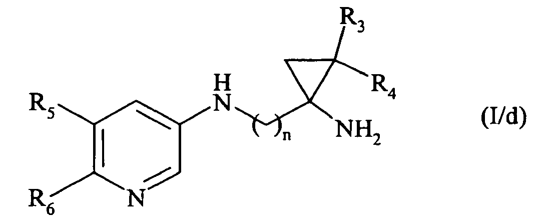
dans laquelle R₃, R₄, R₅, R₆ et n sont tels que définis précédemment,
composés de formule (I/d) qui sont traités par de l'acide formique et une solution aqueuse de formaldéhyde pour conduire aux composés de formule (I/e), cas particulier des composés de formule (I) : 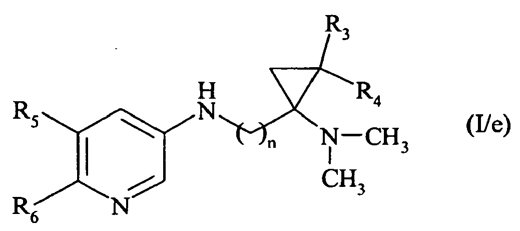 dans laquelle R₃, R₄, R₅, R₆ et n sont tels que définis précédemment,
composés de formules (I/b), (I/c), (I/d) et (I/e) formant les composés de formule (I/f), cas particulier des composés de formule (I) : 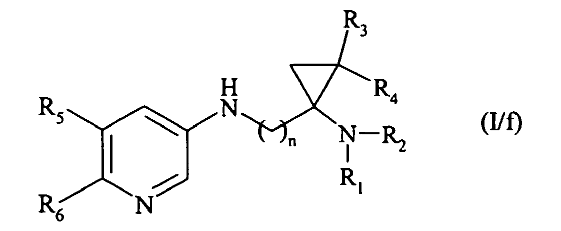 dans laquelle R₁, R₂, R₃, R₄, R₅, R₆ et n sont tels que définis précédemment,
composés de formule (I/f) qui sont mis à réagir avec un composé de formule (XII) :
R'₇-COOH (XII)
dans laquelle R'₇ représente un groupement choisi parmi alkyle (C₁-C₅) linéaire ou ramifié et arylalkyle (C₁-C₅) linéaire ou ramifié, en présence d'un agent de couplage, suivi d'une réduction de l'amide, pour conduire aux composés de formule (I/g), cas particulier des composés de formule (I) : 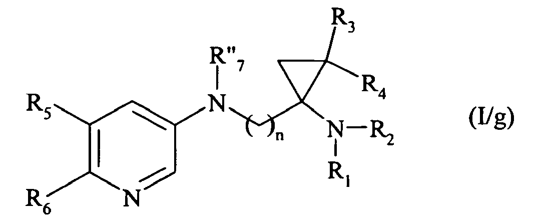 dans laquelle R₁, R₂, R₃, R₄, R₅, R₆ et n sont tels que définis précédemment, et R"₇ représente un groupement choisi parmi alkyle(C₁-C₆) linéaire ou ramifié et arylalkyle (C₁-C₆) linéaire ou ramifié,
- soit mis en présence de carbonyldiimidazole et d'acide formique dans la diméthylformamide pour conduire aux composés de formule (XIII) : 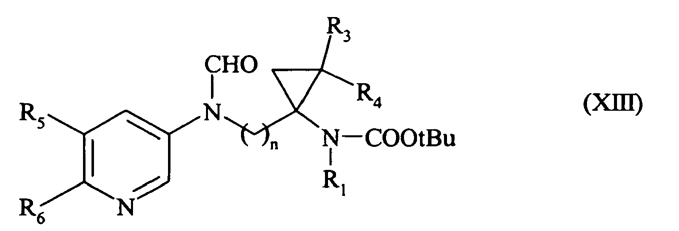
dans laquelle R₁, R₃, R₄, R₅, R₆ et n sont tels que définis précédemment,
composés de formule (XIII) qui sont mis à réagir avec du boraneméthylsulfure complexe dans du tétrahydrofurane pour conduire aux composés de formule (XIV) : 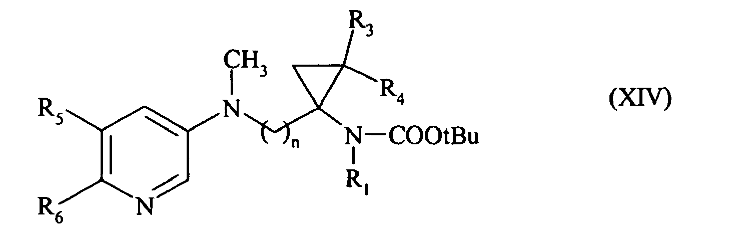 dans laquelle R₁, R₃, R₄, R₅, R₆ et n sont tels que définis précédemment,
composés de formule (XIV) qui sont soumis aux mêmes conditions de réaction que les composés de formule (X), action de l'acide dans le dioxane pour conduire aux composés de formule (I/h), cas particulier des composés de formule (I) : 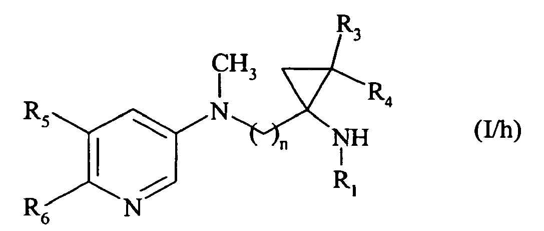 dans laquelle R₁, R₃, R₄, R₅, R₆ et n sont tels que définis précédemment,
composés de formule (I/h) qui sont soumis aux mêmes conditions de réaction que les composés de formule (I/a) lorsque R₁ représente un groupement benzyl, pour conduire aux composés de formule (I/i), cas particuliers des composés de formule (I) : 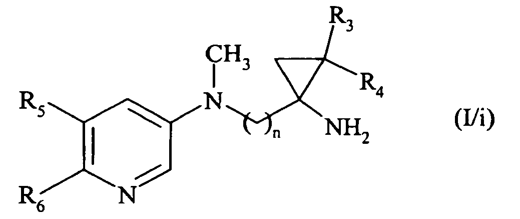 dans laquelle R₃, R₄, R₅, R₆ et n sont tels que définis précédemment, composés de formule (I/i) qui sont soumis aux mêmes conditions de réaction que les composés de formule (I/d), pour conduire aux composés de formule (I/j), cas particuliers des composés de formule (I) : 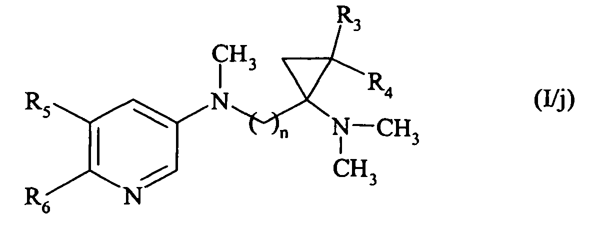 dans laquelle R₃, R₄, R₅, R₆ et n sont tels que définis précédemment,
l'ensemble des composés de formules (I/a) à (I/j) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

9. Compositions pharmaceutiques contenant comme principe actif au moins un composé selon l'une quelconque des revendications 1 à 7, seul ou en combinaison avec un ou plusieurs excipients ou véhicules inertes, non toxiques, pharmaceutiquement acceptables.

10. Compositions pharmaceutiques selon la revendication 9 contenant au moins un principe actif selon l'une quelconque des revendications 1 à 7 utiles comme ligand nicotinique spécifique des récepteurs α4β2.

11. Compositions pharmaceutiques selon la revendication 9 contenant au moins un principe actif selon l'une quelconque des revendications 1 à 7 utiles dans le traitement des déficits de mémoire associés au vieillissement cérébral et aux maladies neurodégénératives, ainsi que pour le traitement des troubles de l'humeur, du syndrome de Tourette, du syndrome d'hyperactivité avec déficits attentionnels, du sevrage tabagique et de la douleur.

12. Compositions pharmaceutiques selon la revendication 9 contenant au moins un principe actif selon l'une quelconque des revendications 1 à 7, utiles dans le traitement des déficits de mémoire associés à la maladie d'Alzheimer, la maladie de Parkinson, la maladie de Pick, la maladie de Korsakoff ou les démences frontales et sous-corticales.

## Claims

1. Compound of formula (I) : 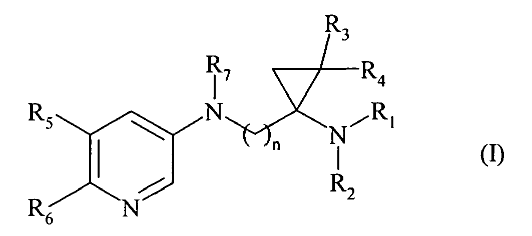 wherein :
n represents an integer of from 1 to 6 inclusive,
R₁ and R₂, which may be identical or different, each independently of the other represent a hydrogen atom, a linear or branched (C₁-C₆)alkyl group or an aryl-(C₁-C₆)alkyl group in which the alkyl moiety may be linear or branched,
R₃ and R₄, which may be identical or different, each independently of the other represent a hydrogen atom or a linear or branched (C₁-C₆)alkyl group,
R₅ and R₆, which may be identical or different, each independently of the other represent a hydrogen atom or a linear or branched (C₁-C₆)alkyl, halogen, hydroxy, linear or branched (C₁-C₆)alkoxy, cyano, nitro, linear or branched (C₂-C₆)acyl, linear or branched (C₁-C₆)alkoxycarbonyl, linear or branched (C₁-C₆)trihaloalkyl or linear or branched (C₁-C₆)trihaloalkoxy group or an amino group optionally substituted by one or two linear or branched (C₁-C₆)alkyl groups,
R₇ represents a hydrogen atom, a linear or branched (C₁-C₆)alkyl group or an aryl-(C₁-C₆)alkyl group in which the alkyl moiety may be linear or branched,
there being understood by aryl group a phenyl, biphenyl, naphthyl, dihydronaphthyl, tetrahydronaphthyl, indanyl or indenyl group, each of those groups being optionally substituted by one or more identical or different groups selected from halogen atoms, linear or branched (C₁-C₆)alkyl, hydroxy, cyano, nitro, linear or branched (C₁-C₆)alkoxy, linear or branched (C₂-C₇)acyl, linear or branched (C₁-C₆)alkoxycarbonyl, linear or branched (C₁-C₆)trihaloalkyl and linear or branched (C₁-C₆)trihaloalkoxy groups, and amino groups optionally substituted by one or two linear or branched (C₁-C₆)alkyl groups, their enantiomers, diastereoisomers, and also addition salts thereof with a pharmaceutically acceptable acid or base.

2. Compounds of formula (I) according to claim 1, **characterised in that** n is an integer having the value 1, their enantiomers, diastereoisomers, and also addition salts thereof with a pharmaceutically acceptable acid or base.

3. Compounds of formula (I) according to claim 1, **characterised in that** R₁ and R₂, which may be identical or different, each independently of the other represent a hydrogen atom or a linear or branched (C₁-C₆)alkyl group, their enantiomers, diastereoisomers, and also addition salts thereof with a pharmaceutically acceptable acid or base.

4. Compounds of formula (I) according to claim 1, **characterised in that** R₃ and R₄ represent a hydrogen atom, their enantiomers, diastereoisomers, and also addition salts thereof with a pharmaceutically acceptable acid or base.

5. Compounds of formula (I) according to claim 1, **characterised in that** R₅ and R₆, which may be identical or different, each independently of the other represent a hydrogen atom or a linear or branched (C₁-C₆)alkyl group, their enantiomers, diastereoisomers, and also addition salts thereof with a pharmaceutically acceptable acid or base.

6. Compounds of formula (I) according to claim 1, **characterised in that** R₇ represents a hydrogen atom or a linear or branched (C₁-C₆)alkyl group, their enantiomers, diastereoisomers, and also addition salts thereof with a pharmaceutically acceptable acid or base.

7. Compounds of formula (I) according to claim 1 which are :
*N*-{[1-(methylamino)cyclopropyl]methyl}pyridin-3-amine
*N*-methyl-*N*-{[1-(methylamino)cyclopropyl]methyl}pyridin-3-amine
*N*-[(1-aminocyclopropyl)methyl]pyridin-3-amine
*N*-{[1-(dimethylamino)cyclopropyl]methyl}pyridin-3-amine
*N*-[(1-aminocyclopropyl)methyl]-*N*-methylpyridin-3-amine
*N*-{[1-(dimethylamino)cyclopropyl]methyl}-*N*-methylpyridin-3-amine
6-chloro-*N*-{[1-(methylamino)cyclopropyl]methyl}pyridin-3-amine
6-chloro-*N*-methyl-*N*-{[1-(methylamino)cyclopropyl]methyl}pyridin-3-amine
6-bromo-*N*-{[1-(methylamino)cyclopropyl]methyl}pyridin-3-amine
6-bromo-*N*-methyl-*N*-{[1-(methylamino)cyclopropyl]methyl}pyridin-3-amine
6-methyl-*N*-{[1-(methylamino)cyclopropyl]methyl}pyridin-3-amine
*N*,6-dimethyl-*N*-{[1-(methylamino)cyclopropyl]methyl}pyridin-3-amine,
their enantiomers, diastereoisomers, and also addition salts thereof with a pharmaceutically acceptable acid or base.

8. Process for the preparation of compounds of formula (I) according to claim 1, **characterised in that** there is used as starting material a compound of formula (II) : 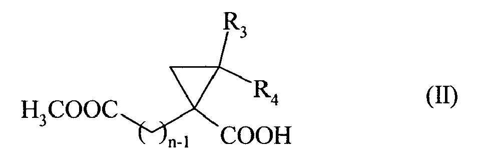 wherein R₃, R₄ and n are as defined for formula (I),
which compounds of formula (II) are reacted with diphenylphosphoryl azide in the presence of triethyleneamine in toluene, followed by the addition of *tert*-butanol, to yield compounds of formula (III) : 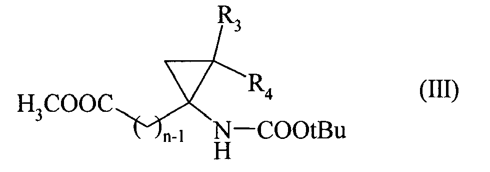 wherein R₃, R₄ and n are as defined above, which compounds of formula (III) are reacted with a compound of formula (IV) :
R'₁ - Hal (IV)
wherein Hal represents a halogen atom and R'₁ represents a group selected from linear or branched (C₁-C₆)alkyl and aryl-(C₁-C₆)alkyl in which the alkyl moiety may be linear or branched, in the presence of a base in an anhydrous solvent, to yield compounds of formula (V) : 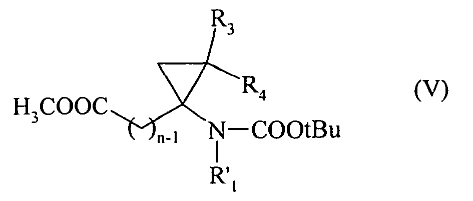 wherein R₃, R₄, n and R'₁ are as defined above,
the compounds of formulae (III) and (V) constituting the compounds of formula (VI) : 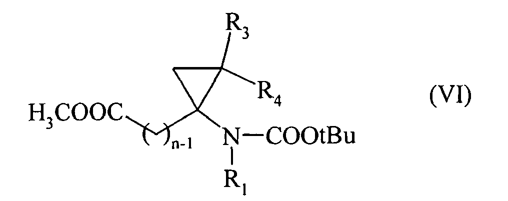 wherein R₃, R₄ and n are as defined above and R₁ is as defined for formula (I),
which compounds of formula (VI) are placed in the presence of a reducing agent to yield compounds of formula (VII) : 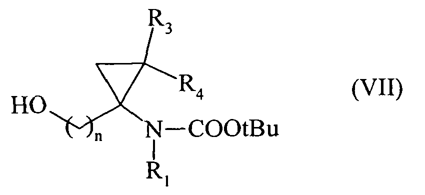 wherein R₃, R₄, n and R₁ are as defined above,
which compounds of formula (VII) are subjected to the action of an oxidising agent conventional in organic synthesis to yield compounds of formula (VIII) : 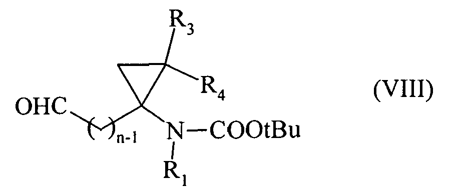 wherein R₃, R₄, n and R₁ are as defined above,
which compounds of formula (VIII) are reacted with a compound of formula (IX) : 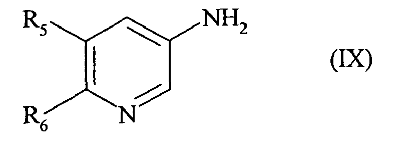 wherein R₅ and R₆ are as defined for formula (I), in the presence of sodium triacetoxyborohydride, to yield compounds of formula (X) : 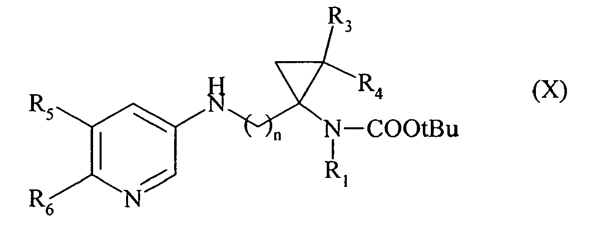 wherein R₃, R₄, R₅, R₆, n and R₁ are as defined above,
which compounds of formula (X) are :
- either placed in the presence of an acid in dioxane to yield compounds of formula (I/a), a particular case of the compounds of formula (I) : 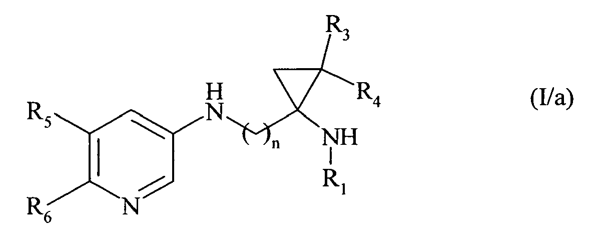
wherein R₁, R₃, R₄, R₅, R₆ and n are as defined above,
which compounds of formula (I/a) are :
- either placed in the presence of formol and formic acid to yield compounds of formula (I/b), a particular case of the compounds of formula (I) : 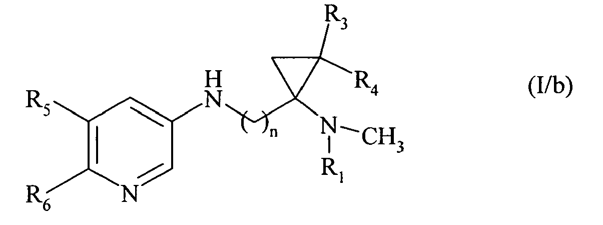
wherein R₁, R₃, R₄, R₅, R₆ and n are as defined above,
- or reacted with a compound of formula (XI) :
R'₂ - Hal (XI)
wherein Hal is as defined above and R'₂ represents a group selected from linear or branched (C₁-C₆)alkyl and aryl-(C₁-C₆)alkyl in which the alkyl moiety may be linear or branched, in the presence of a base in an anhydrous solvent, to yield compounds of formula (I/c), a particular case of the compounds of formula (I) : 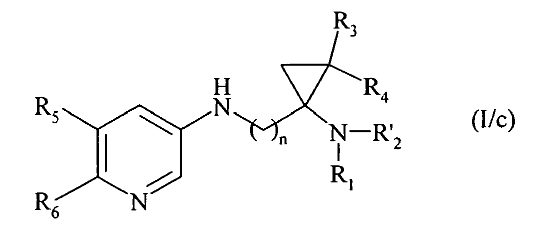
wherein R₁, R₃, R₄, R₅, R₆, n and R'₂ are as defined above,
- or, when R₁ represents a benzyl group, placed in the presence of hydrochloric acid and palladium-on-carbon to yield compounds of formula (I/d), a particular case of the compounds of formula (I) : 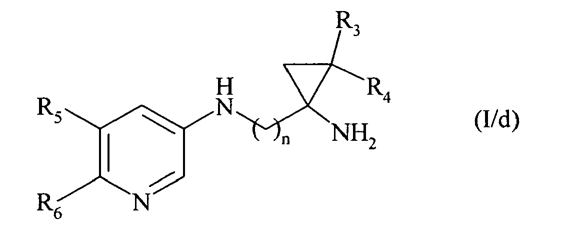
wherein R₃, R₄, R₅, R₆ and n are as defined above,
which compounds of formula (I/d) are treated with formic acid and an aqueous formaldehyde solution to yield compounds of formula (I/e), a particular case of the compounds of formula (I) : 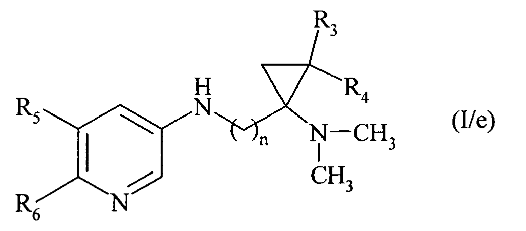 wherein R₃, R₄, R₅, R₆ and n are as defined above,
the compounds of formulae (I/b), (I/c), (I/d) and (I/e) constituting the compounds of formula (I/f), a particular case of the compounds of formula (I) : 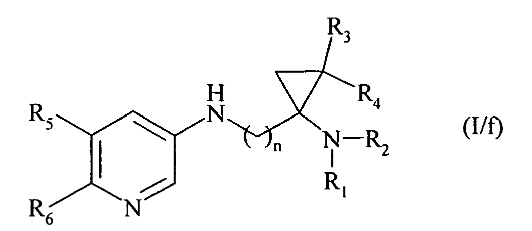 wherein R₁, R₂, R₃, R₄, R₅, R₆ and n are as defined above,
which compounds of formula (I/f) are reacted with a compound of formula (XII) :
R'₇ - COOH (XII),
wherein R'₇ represents a group selected from linear or branched (C₁-C₅)alkyl and aryl-(C₁-C₅)alkyl in which the alkyl moiety may be linear or branched, in the presence of a coupling agent, followed by reduction of the amide, to yield compounds of formula (I/g), a particular case of the compounds of formula (I) : 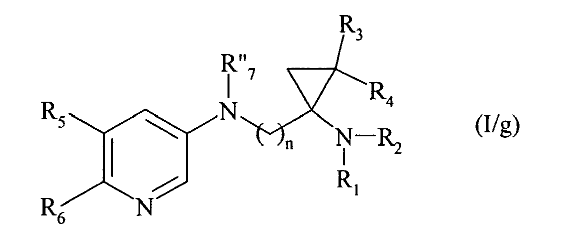 wherein R₁, R₂, R₃, R₄, R₅, R₆ and n are as defined above and R"₇ represents a group selected from linear or branched (C₁-C₆)alkyl and aryl-(C₁-C₆)alkyl in which the alkyl moiety may be linear or branched,
- or placed in the presence of carbonyldiimidazole and formic acid in dimethylformamide to yield compounds of formula (XIII) : 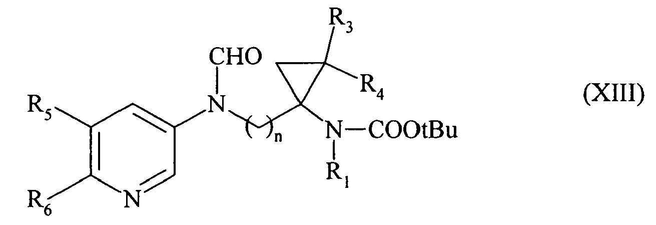
wherein R₁, R₃, R₄, R₅, R₆ and n are as defined above,
which compounds of formula (XIII) are reacted with borane-methyl sulphide complex in tetrahydrofuran to yield compounds of formula (XIV) : 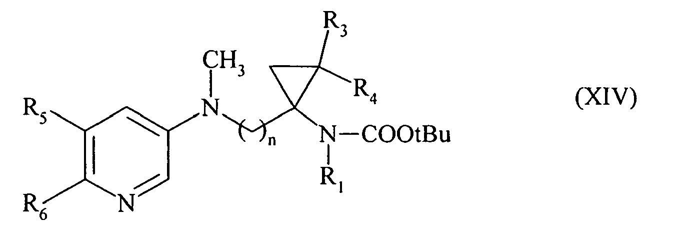 wherein R₁, R₃, R₄, R₅, R₆ and n are as defined above,
which compounds of formula (XIV) are subjected to the same reaction conditions as the compounds of formula (X), action of the acid in dioxane, to yield compounds of formula (I/h), a particular case of the compounds of formula (I) : 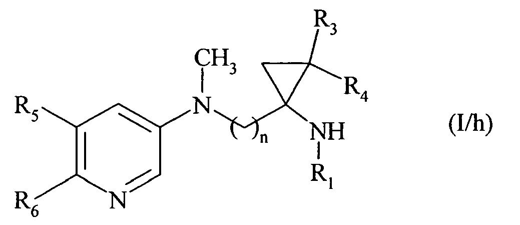 wherein R₁, R₃, R₄, R₅, R₆ and n are as defined above,
which compounds of formula (I/h), when R₁ represents a benzyl group, are subjected to the same reaction conditions as the compounds of formula (I/a) to yield compounds of formula (I/i), particular cases of the compounds of formula (I) : 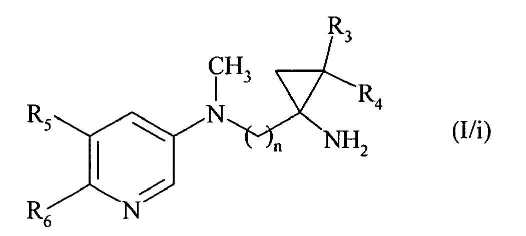 wherein R₃, R₄, R₅, R₆ and n are as defined above, which compounds of formula (I/i) are subjected to the same reaction conditions as the compounds of formula (I/d) to yield compounds of formula (I/j), particular cases of the compounds of formula (I) : 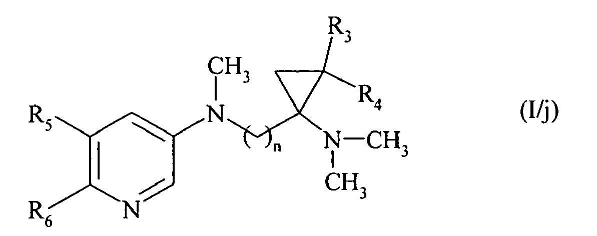 wherein R₃, R₄, R₅, R₆ and n are as defined above,
the totality of the compounds of formulae (I/a) to (I/j) constituting the totality of the compounds of the invention, which are purified, where appropriate, according to conventional purification techniques, which may be separated into their different isomers according to a conventional separation technique, and which are converted, where appropriate, into addition salts thereof with a pharmaceutically acceptable acid or base.

9. Pharmaceutical compositions comprising as active ingredient at least one compound according to any one of claims 1 to 7, alone or in combination with one or more pharmaceutically acceptable, inert, non-toxic excipients or carriers.

10. Pharmaceutical compositions according to claim 9 comprising at least one active ingredient according to any one of claims 1 to 7 for use as a specific nicotinic ligand of α4β2 receptors.

11. Pharmaceutical compositions according to claim 9 comprising at least one active ingredient according to any one of claims 1 to 7 for use in the treatment of deficiencies of memory associated with cerebral ageing and with neurodegenerative diseases, and also for the treatment of mood disorders, Tourette's syndrome, attention-deficit hyperactivity syndrome, tobacco withdrawal and pain.

12. Pharmaceutical compositions according to claim 9 comprising at least one active ingredient according to any one of claims 1 to 7 for use in the treatment of deficiencies of memory associated with Alzheimer's disease, Parkinson's disease, Pick's disease, Korsakoff's disease or frontal lobe and subcortical dementias.

## Patentansprüche

1. Verbindungen der Formel (I): 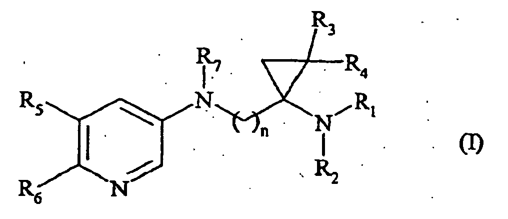 in der:
n eine ganze Zahl mit einem Wert zwischen 1 und 6 einschließlich bedeutet,
R₁ und R₂, die gleichartig oder verschieden sind, unabhängig voneinander ein Wasserstoffatom oder eine geradkettige oder verzweigte (C₁-C₆)-Alkylgruppe oder eine geradkettige oder verzweigte Aryl-(C₁-C₆)-alkylgruppe bedeuten,
R₃ und R₄, die gleichartig oder verschieden sind, unabhängig voneinander ein Wasserstoffatom oder eine geradkettige oder verzweigte (C₁-C₆)Alkylgruppe bedeuten,
R₅ und R₆, die gleichartig oder verschieden sind, unabhängig voneinander ein Wasserstoffatom oder eine geradkettige oder verzweigte (C₁-C₆)-Alkylgruppe, ein Halogenatom, eine Hydroxygruppe, eine geradkettige oder verzweigte (C₁-C₆)-Alkoxygruppe, eine Cyanogruppe, Nitrogruppe, geradkettige oder verzweigte (C₂-C₆)-Acylgruppe, geradkettige oder verzweigte (C₁-C₆)-Alkoxycarbonylgruppe, geradkettige oder verzweigte (C₁-C₆)-Trihalogenalkylgruppe, geradkettige oder verzweigte (C₁-C₆)-Trihalogenalkoxygruppe oder eine gegebenenfalls durch eine oder zwei geradkettige oder verzweigte (C₁-C₆)-Alkylgruppe substituierte Aminogruppe bedeuten, und
R₇ ein Wasserstoffatom oder eine geradkettige oder verzweigte (C₁-C₆)-Alkylgruppe oder eine geradkettige oder verzweigte Aryl-(C₁-C₆)-alkylgruppe bedeutet,
mit der Maßgabe, dass man unter einer Arylgruppe eine Phenyl-, Biphenyl-, Naphthyl-, Dihydronaphthyl-, Tetrahydronaphthyl-, Indanyl- und Indenyl-Gruppe versteht, wobei jede dieser Gruppen gegebenenfalls durch eine oder mehrere gleichartige oder verschiedenartige Gruppen substituiert ist, ausgewählt aus Halogen, geradkettigem oder verzweigtem (C₁-C₆)-Alkyl, Hydroxy, Cyano, Nitro, geradkettigem oder verzweigtem (C₁-C₆)-Alkoxy, geradkettigem oder verzweigtem (C₂-C₇)-Acyl, geradkettigem oder verzweigtem (C₁-C₆)-Alkoxycarbonyl, geradkettigem oder verzweigtem (C₁-C₆)-Trihalogenalkyl, geradkettigem oder verzweigtem (C₁-C₆)-Trihalogenalkoxy und Amino, welches gegebenenfalls durch eine oder zwei geradkettige oder verzweigte (C₁-C₆)-Alkylgruppen substituiert ist, deren Enantiomere, Diastereoisomere sowie deren Additionssalze mit einer pharmazeutisch annehmbaren Säure oder Base.

2. Verbindungen der Formel (I) nach Anspruch 1, **dadurch gekennzeichnet, dass** n eine ganze Zahl mit einem Wert von 1 besitzt, deren Enantiomere, Diastereoisomere sowie deren Additionssalze mit einer pharmazeutisch annehmbaren Säure oder Base.

3. Verbindungen der Formel (I) nach Anspruch 1, **dadurch gekennzeichnet, dass** R₁ und R₂, die gleichartig oder verschieden sind, unabhängig voneinander ein Wasserstoffatom oder eine geradkettige oder verzweigte (C₁-C₆)-Alkylgruppe bedeuten, deren Enantiomere, Diastereoisomere sowie deren Additionssalze mit einer pharmazeutisch annehmbaren Säure oder Base.

4. Verbindungen der Formel (I) nach Anspruch 1, **dadurch gekennzeichnet, dass** R₃ und R₄ ein Wasserstoffatom bedeuten, deren Enantiomere, Diastereoisomere sowie deren Additionssalze mit einer pharmazeutisch annehmbaren Säure oder Base.

5. Verbindungen der Formel (I) nach Anspruch 1, **dadurch gekennzeichnet, dass** R₅ und R₆, die gleichartig oder verschieden sind, unabhängig voneinander ein Wasserstoffatom oder eine geradkettige oder verzweigte (C₁-C₆)-Alkylgruppe bedeuten, deren Enantiomere, Diastereoisomere sowie deren Additionssalze mit einer pharmazeutisch annehmbaren Säure oder Base.

6. Verbindungen der Formel (I) nach Anspruch 1, **dadurch gekennzeichnet, dass** R₇ ein Wasserstoffatom oder eine geradkettige oder verzweigte (C₁-C₆)-Alkylgruppe bedeutet, deren Enantiomere, Diastereoisomere sowie deren Additionssalze mit einer pharmazeutisch annehmbaren Säure oder Base.

7. Verbindungen der Formel (I) nach Anspruch 1, nämlich:
*N*-{[1-(Methylamino)-cyclopropyl]-methyl}-pyridin-3-amin
*N*-Methyl-N-{[1-(methylamino)-cyclopropyl]-methyl}-pyridin-3-amin
*N*-[(1-Aminocyclopropyl)-methyl]-pyridin-3-amin
*N*-{[1-(Dimethylamino)-cyclopropyl]-methyl}-pyridin-3-amin
*N*-[(1-Aminocyclopropyl)-methyl]-*N*-methylpyridin-3-amin
*N*-{[1-(Dimethylamino)-cyclopropyl]-methyl}-*N*-methylpyridin-3-amin
6-Chlor-*N*-{[1-(methylamino)-cyclopropyl]-methyl}-pyridin-3-amin
6-Chlor-*N*-methyl-*N*-{[1-(methylamino)-cyclopropyl]-methyl}-pyridin-3-amin
6-Brom-*N*-{[1-(methylamino)-cyclopropyl]-methyl}-pyridin-3-amin
6-Brom-*N*-methyl-*N*-{[1-(methylamino)-cyclopropyl]-methyl}-pyridin-3-amin
6-Methyl-*N*-{[1-(methylamino)-cyclopropyl]-methyl}-pyridin-3-amin
*N*,6-Dimethyl-*N*-{[1-(methylamino)-cyclopropyl]-methyl}-pyridin-3-amin
deren Enantiomere, Diastereoisomere sowie deren Additionssalze mit einer pharmazeutisch annehmbaren Säure oder Base.

8. Verfahren zur Herstellung der Verbindungen der Formel (I) nach Anspruch 1, **dadurch gekennzeichnet, dass** man als Ausgangsprodukt eine Verbindung der Formel (II) verwendet: 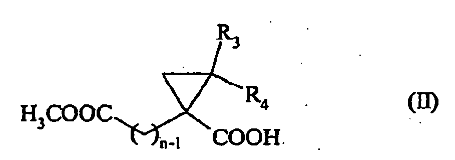 in der R₃, R₄ und n die bezüglich der Formel (I) angegebenen Bedeutungen besitzen,
welche Verbindungen der Formel (II) man mit Diphenylphosphorylazid in Gegenwart von Triethylamin in Toluol umsetzt, gefolgt von der Zugabe von tert.-Butanol zur Bildung der Verbindungen der Formel (III): 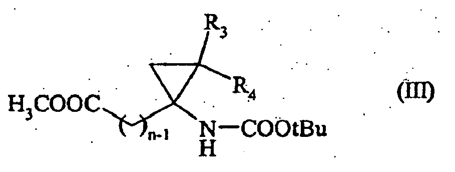 in der R₃, R₄ und n die oben angegebenen Bedeutungen besitzen, welche Verbindungen der Formel (III) man mit einer Verbindung der Formel (IV):
R'₁-Hal (IV)
in der Hal ein Halogenatom und R'₁ eine Gruppe ausgewählt aus geradkettigem oder verzweigtem (C₁-C₆)-Alkyl und geradkettigem oder verzweigtem Aryl-(C₁-C₆)-alkyl in Gegenwart einer Base in einem wasserfreien Lösungsmittel umsetzt zur Bildung der Verbindungen der Formel (V): 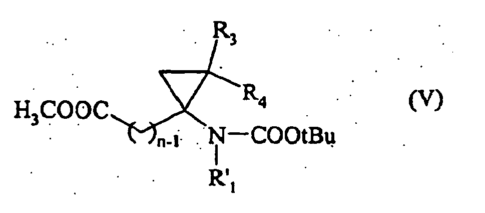 in der R"₃, R₄, n und R'₁ die oben angegebenen Bedeutungen besitzen,
welche Verbindungen der Formeln (III) und (V) die Verbindungen der Formel (VI) bilden: 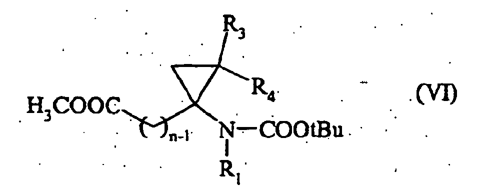 in der R₃, R₄ und n die oben angegebenen Bedeutungen besitzen und R₁ die bezüglich der Formel (I) angegebenen Bedeutungen aufweist,
welche Verbindungen der Formel (VI) man mit einem Reduktionsmittel umsetzt zur Bildung der Verbindungen der Formel (VII): 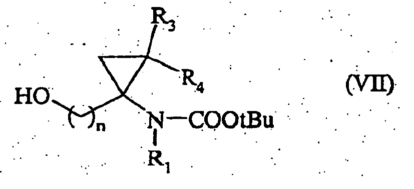 in der R₃, R₄, n und R₁ die oben angegebenen Bedeutungen besitzen,
welche Verbindungen der Formel (VII) man der Einwirkung eines in der organischen Synthese klassischen Oxidationsmittels unterwirft zur Bildung der Verbindungen der Formel (VIII): 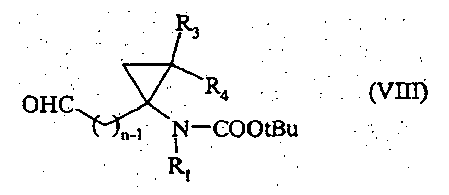 in der R₃, R₄, n und R₁ die oben angegebenen Bedeutungen besitzen,
welche Verbindungen der Formel (VIII) man mit einer Verbindung der Formel (IX): 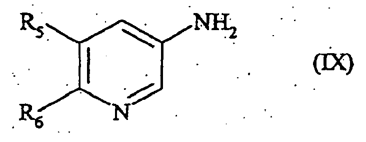 in der R₅ und R₆ die bezüglich der Formel (I) angegebenen Bedeutungen besitzen, in Gegenwart von Natriumtriacetoxyborhydrid umsetzt zur Bildung der Verbindungen der Formel (X): 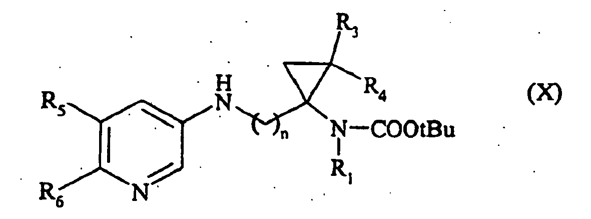 in der R₃, R₄, R₅, R₆, n und R₁ die oben angegebenen Bedeutungen besitzen, welche Verbindungen der Formel (X) man:
- entweder mit einer Säure in Dioxan umsetzt zur Bildung der Verbindungen der Formel (I/a), einem Sonderfall der Verbindungen der Formel (I): 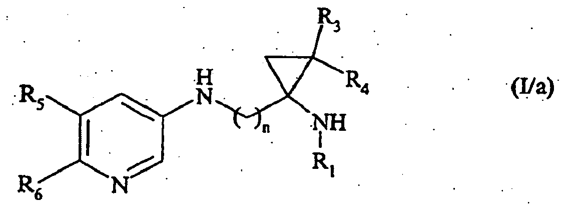 in der R₁, R₃, R₄, R₅, R₆ und n die oben angegebenen Bedeutungen besitzen, welche Verbindungen der Formel (I/a):
- oder mit Formaldehyd und Ameisensäure umsetzt zur Bildung der Verbindungen der Formel (I/b), einem Sonderfall der Verbindungen der Formel (I): 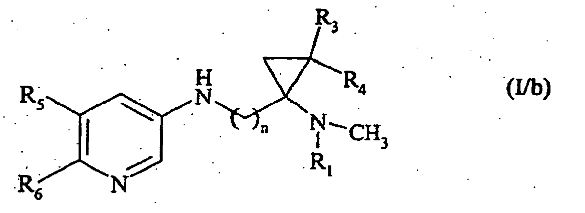 in der R₁, R₃, R₄, R₅, R₆ und n die oben angegebenen Bedeutungen besitzen,
- oder mit einer Verbindung der Formel (XI):
R'₂-Hal (XI)
in der Hal die oben angegebene Bedeutung besitzt und R'₂ eine Gruppe ausgewählt aus geradkettigem oder verzweigtem (C₁-C₆)-Alkyl und geradkettigem oder verzweigtem Aryl-(C₁-C₆)-alkyl bedeutet, in Gegenwart einer Base in einem wasserfreiem Lösungsmittel umsetzt zur Bildung der Verbindungen der Formel (I/c), einem Sonderfall der Verbindungen der Formel (I): 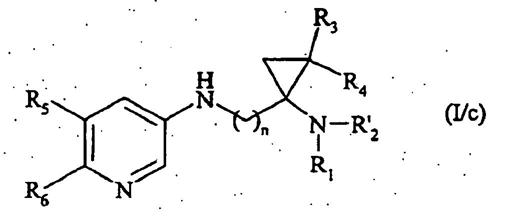 in der R₁, R₃, R₄, R₅, R₆, n und R'₂ die oben angegebenen Bedeutungen besitzen,
- oder, wenn R₁ eine Benzylgruppe darstellt, mit Chlorwasserstoffsäure und Palladium-auf-Kohlenstoff umsetzt zur Bildung der Verbindungen der Formel (I/d), einem Sonderfall der Verbindungen der Formel (I): 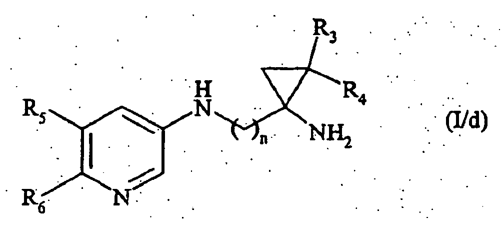 in der R₃, R₄, R₅, R₆ und n die oben angegebenen Bedeutungen besitzen,
welche Verbindungen der Formel (I/d) mit Ameisensäure und einer wässrigen Formaldehydlösung behandelt werden zur Bildung der Verbindungen der Formel (I/e), einem Sonderfall der Verbindungen der Formel (I): 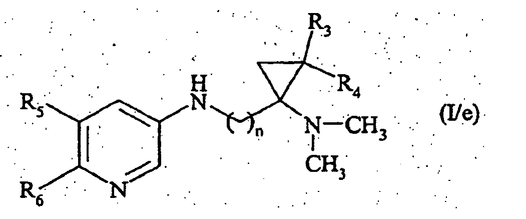 in der R₃, R₄, R₅, R₆ und n die oben angegebenen Bedeutungen besitzen,
welche Verbindungen der Formeln (I/b), (I/c), (I/d) und (I/e) die Verbindungen der Formel (I/f) bilden, einem Sonderfall der Verbindungen der Formel (I): 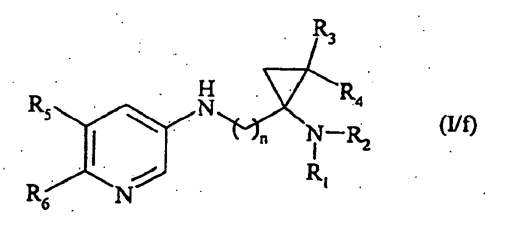 in der R₁, R₂, R₃, R₄, R₅, R₆ und n die oben angegebenen Bedeutungen besitzen, welche Verbindungen der Formel (I/f) mit einer Verbindung der Formel (XII):
R'₇-COOH (XII)
in der R'₇ eine Gruppe bedeutet ausgewählt aus geradkettigem oder verzweigtem (C₁-C₅)-Alkyl und geradkettigem oder verzweigtem Aryl-(C₁-C₅)-alkyl in Gegenwart eines Kupplungsmittels umgesetzt werden gefolgt von einer Reduktion des Amids zur Bildung der Verbindungen der Formel (I/g), einem Sonderfall der Verbindungen der Formel (I): 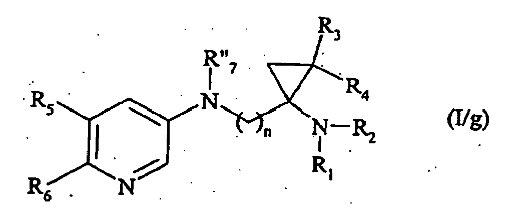 in der R₁, R₂, R₃, R₄, R₅, R₆ und n die oben angegebenen Bedeutungen besitzen und R"₇ eine Gruppe bedeutet ausgewählt aus geradkettigem oder verzweigtem (C₁-C₆)-Alkyl und geradkettigem oder verzweigtem Aryl-(C₁-C₆)-alkyl,
- oder mit Carbonyldiimidazol und Ameisensäure in Dimethylformamid umsetzt zur Bildung der Verbindungen der Formel (XIII): 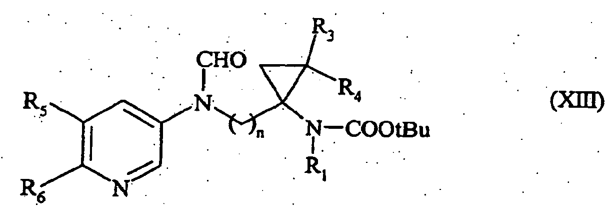 in der R₁, R₃, R₄, R₅, R₆ und n die oben angegebenen Bedeutungen besitzen,
welche Verbindungen der Formel (XIII) mit dem Boranmethylsulfid-Komplex in Tetrahydrofuran umgesetzt werden zur Bildung der Verbindungen der Formel (XIV): 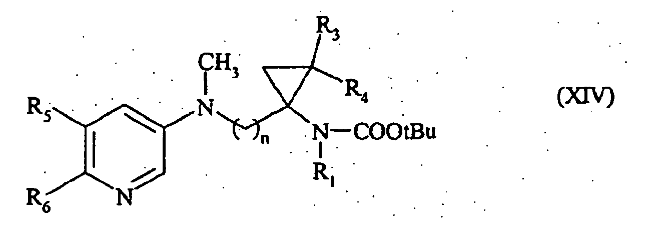 in der R₁, R₃, R₄, R₅, R₆ und n die oben angegebenen Bedeutungen besitzen,
welche Verbindungen der Formel (XIV) den gleichen Reaktionsbedingungen unterworfen werden wie die Verbindungen der Formel (X) und der Einwirkung einer Säure in Dioxan zur Bildung der Verbindungen der Formel (I/h), einem Sonderfall der Verbindungen der Formel (I): 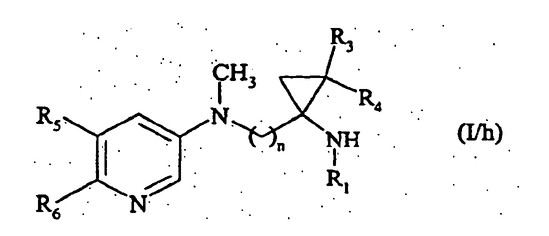 in der R₁, R₃, R₄, R₅, R₆ und n die oben angegebenen Bedeutungen besitzen,
welche Verbindungen der Formel (I/h) den gleichen Reaktionsbedingungen unterworfen werden wie die Verbindungen der Formel (I/a), wenn R₁ eine Benzylgruppe bedeutet, zur Bildung der Verbindungen der Formel (I/i), einem Sonderfall der Verbindungen der Formel (I): 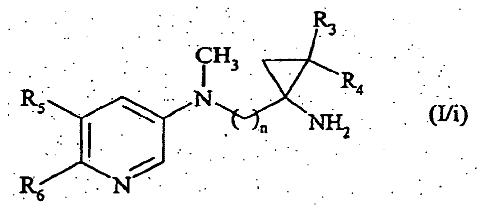 in der R₃, R₄, R₅, R₆ und n die oben angegebenen Bedeutungen besitzen, welche Verbindungen der Formel (I/i) den gleichen Reaktionsbedingungen unterworfen werden wie die Verbindungen der Formel (I/d), zur Bildung der Verbindungen der Formel (I/j), einem Sonderfall der Verbindungen der Formel (I): 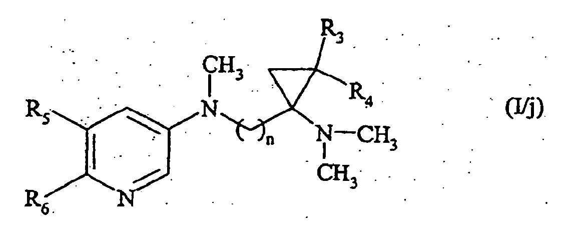 in der R₃, R₄, R₅, R₆ und n die oben angegebenen Bedeutungen besitzen,
wobei die Gesamtheit der Verbindungen der Formeln (I/a) bis (I/j) die Gesamtheit der erfindungsgemäßen Verbindungen bilden, die gegebenenfalls mit Hilfe klassischer Reinigungsmethoden gereinigt werden, die mit Hilfe einer klassischen Trennmethode in ihre verschiedenen Isomeren aufgetrennt werden und die gegebenenfalls in ihre Additionssalze mit einer pharmazeutisch annehmbaren Säure oder Base überführt werden können.

9. Pharmazeutische Zubereitungen, enthaltend als Wirkstoff mindestens eine Verbindung nach einem der Ansprüche 1 bis 7, allein oder in Kombination mit einem oder mehreren inerten, nichttoxischen, pharmazeutisch annehmbaren Hilfsstoffen oder Trägermaterialien.

10. Pharmazeutische Zubereitungen nach Anspruch 9, enthaltend mindestens einen Wirkstoff nach einem der Ansprüche 1 bis 7, nützlich als spezifischer Nicotin-Ligand der Rezeptoren α4β2.

11. Pharmazeutische Zubereitungen nach Anspruch 9, enthaltend mindestens einen Wirkstoff nach einem der Ansprüche 1 bis 7, die nützlich sind bei der Behandlung von Gedächtnisstörungen, die mit dem Altern des Gehirns und mit neurodegenerativen Erkrankungen verknüpft sind, sowie zur Behandlung von Gemütsstörungen, des Tourette-Syndroms, des Hyperaktivitätssyndroms mit Aufmerksamkeitsdefiziten, des Tabakentzugs und von Schmerzen.

12. Pharmazeutische Zubereitungen nach Anspruch 9, enthaltend mindestens einen Wirkstoff nach einem der Ansprüche 1 bis 7, die nützlich sind bei der Behandlung von Gedächtnisdefiziten, die mit der Alzheimerschen Krankheit, der Parkinsonschen Krankheit, der Pickschen Krankheit, der Korsakoff-Krankheit oder frontalen und subkortikalen Demenzien verknüpft sind.
